# EUROPEAN PATENT SPECIFICATION

(11) **EP 3 745 951 B1**
(45) Date of publication and mention of the grant of the patent: **25.03.2026**
(21) Application number: 19746801.0
(22) Date of filing: 01.02.2019
(51) Int. Cl.: A61B 5/00, A61K 49/00, G06T 7/90, G16H 50/20

(54) **DEVICES, SYSTEMS, AND METHODS FOR TUMOR VISUALIZATION AND REMOVAL**
VORRICHTUNGEN, SYSTEME UND VERFAHREN ZUR TUMORVISUALISIERUNG UND -ENTFERNUNG
DISPOSITIFS, SYSTÈMES ET PROCÉDÉS POUR LA VISUALISATION ET L'ÉLIMINATION DE TUMEURS

(30) Priority: 02.02.2018 US 201862625967 P; 03.02.2018 US 201862625983 P; 17.01.2019 US 201962793843 P
(43) Date of publication of application: 09.12.2020
(73) Proprietor: University Health Network, Toronto, ON M5G 2C4 (CA)
(72) Inventor: DACOSTA, Ralph, Etobicoke, ON M9A 3B9 (CA); GIBSON, Christopher, Toronto, ON M4Y1M2 (CA); OTTOLINO-PERRY, Kathryn, Toronto, ON M6S 2M7 (CA); ANANTHA, Nayana, Thalanki, Scarborough, ON M1K 3Y4 (CA); DONE, Susan, Jane, Toronto, ON M4R 1M9 (CA); LEONG, Wey-Liang, Toronto, ON M5P 1B1 (CA); EASSON, Alexandra M., Toronto, ON M4R 1J8 (CA)
(74) Representative: PGA S.p.A., Milano, Succursale di Lugano
(86) International application number: PCT/CA2019/000015
(87) International publication number: WO 2019/148268

(56) References cited:
- US-A1- 2006 004 292
- US-A1- 2014 276 102
- US-A1- 2016 045 114
- US-A1- 2017 290 515
- TADANOBU NAGAYA ET AL: "Fluorescence-Guided Surgery", FRONTIERS IN ONCOLOGY, vol. 7, 22 December 2017 (2017-12-22), XP055580138, DOI: 10.3389/fonc.2017.00314
- GAO ET AL.: "Fluroscent chemical probes for accurate tumor diagnosis and targeting therapy", FLUORESCENT CHEMICAL PROBES FOR ACCURAT E TUMOR DIAGNOSIS AND TARGETINE THERAPY, vol. 46, no. 8, 2017, pages 2237 - 2271, XP055628295, ISSN: 0306-0012, DOI: 10.1039/C6CS00908E
- NTZIACHRISTOS ET AL.: "Current concepts and future perspectives on surgical optical imaging in cancer", JOURNAL OF BIOMEDICAL OPTICS, 2010, pages 66024, XP055354007

## Description

### TECHNICAL FIELD

The present disclosure relates to devices, systems, and methods for tumor visualization and removal. The disclosed devices, systems, and methods may also be used to stage tumors and to assess surgical margins and specimens such as tissue margins, excised tissue specimens, and tissue slices of excised tumors and margins on tissue beds/surgical beds from which a tumor and/or tissue has been removed. The disclosed devices, systems, and methods may also be used to identify one or more of residual cancer cells, precancerous cells, and satellite lesions and to provide guidance for removal and/or treatment of the same. The disclosed devices may be used to obtain materials to be used for diagnostic and planning purposes.

### INTRODUCTION

Surgery is one of the oldest types of cancer therapy and is an effective treatment for many types of cancer. Oncology surgery may take different forms, dependent upon the goals of the surgery. For example, oncology surgery may include biopsies to diagnose or determine a type or stage of cancer, tumor removal to remove some or all of a tumor or cancerous tissue, exploratory surgery to locate or identify a tumor or cancerous tissue, debulking surgery to reduce the size of or remove as much of a tumor as possible without adversely affecting other body structures, and palliative surgery to address conditions caused by a tumor such as pain or pressure on body organs.

In surgeries in which the goal is to remove the tumor(s) or cancerous tissue, surgeons often face uncertainty in determining if all cancer has been removed. The surgical bed, or tissue bed, from which a tumor is removed, may contain residual cancer cells, i.e., cancer cells that remain in the surgical margin of the area from which the tumor is removed. If these residual cancer cells remain in the body, the likelihood of recurrence and metastasis increases. Often, the suspected presence of the residual cancer cells, based on examination of surgical margins of the excised tissue during pathological analysis of the tumor, leads to a secondary surgery to remove additional tissue from the surgical margin.

For example, breast cancer, the most prevalent cancer in women, is commonly treated by breast conservation surgery (BCS), e.g., a lumpectomy, which removes the tumor while leaving as much healthy breast tissue as possible. Treatment efficacy of BCS depends on the complete removal of malignant tissue while leaving enough healthy breast tissue to ensure adequate breast reconstruction, which may be poor if too much breast tissue is removed. Visualizing tumor margins under standard white light (WL) operating room conditions is challenging due to low tumor-to-normal tissue contrast, resulting in reoperation (i.e., secondary surgery) in approximately 23% of patients with early stage invasive breast cancer and 36% of patients with ductal carcinoma *in situ.* Re-excision is associated with a greater risk of recurrence, poorer patient outcomes including reduced breast cosmesis and increased healthcare costs. Positive surgical margins (i.e., margins containing cancerous cells) following BCS are also associated with decreased disease specific survival.

<Document US20060004292A1 discloses a method and an apparatus for enhancing the optical detection of target portions of an object, particularly useful in the optical examination of biological tissue to distinguish cancerous tissue from non-cancerous tissue.

Document US20140276102A1 discloses embodiments related to medical imaging devices for identifying abnormal tissue, such as cancer cells, within a surgical bed. Document XP55580138A titled "Fluorescence-Guided Surgery" is a scientific paper disclosing fluorescent probes that may assist Fluorescence-guided surgery (FGS).>

Current best practice in BCS involves palpation and/or specimen radiography and rarely, intraoperative histopathology to guide resection. Specimen radiography evaluates excised tissue margins using x-ray images and intraoperative histopathology (touch-prep or frozen) evaluates small samples of specimen tissue for cancer cells, both of which are limited by the time delay they cause (~ 20 min) and inaccurate colocalization of a positive margin on the excised tissue to the surgical bed. Thus, there is an urgent clinical need for a real-time, intraoperative imaging technology to assess excised specimen and surgical bed margins and to provide guidance for visualization and removal of one or more of residual cancer cells, precancerous cells, and satellite lesions.

### SUMMARY

A handheld device according to the present invention is disclosed in claims 1-13. A multispectral system according to the present invention is disclosed in claim 14. A kit according to the present invention is disclosed in claims 15-17.

A method according to the present invention is disclosed in claim 18.

In accordance with another aspect of the present disclosure, a method of visualizing a tissue of interest in a patient is disclosed. The method comprises administering to the patient, in a diagnostic dosage, a non-activated, non-targeted compound configured to induce porphyrins in cancerous tissue. The method further comprises, between about 15 minutes and about 6 hours after administering the compound, removing tissue containing the induced porphyrins from the patient, wherein removing the tissue creates a surgical cavity. The method also includes, with a handheld white light and fluorescence-based imaging device, viewing a surgical margin of at least one of the removed tissue cells, one or more sections of the removed tissue cells, and the surgical cavity to visualize any induced porphyrins contained in tissues of the surgical margin.

In accordance with yet another aspect of the present disclosure, a handheld, white light and fluorescence-based imaging device for visualizing at least one of precancerous cells, cancerous cells, and satellite lesions in surgical margins is disclosed. The device comprises a body having a first end portion configured to be held in a user's hand and a second end portion configured to direct light onto a surgical margin. The body contains at least one excitation light source configured to excite autofluorescence emissions of tissue cells and fluorescence emissions of induced porphyrins in tissue cells of the surgical margin, The body also contains a filter configured to prevent passage of reflected excitation light and permit passage of emissions having a wavelength corresponding to autofluorescence emissions of tissue cells and fluorescence emissions of the induced porphyrins in tissue cells. The body further contains an imaging lens, an image sensor configured to detect the filtered autofluorescence emissions of tissue cells and fluorescence emissions of the induced porphyrins in tissue cells of the surgical margin, and a processor configured to receive the detected emissions and to output data regarding the detected filtered autofluorescence emissions of tissue cells and fluorescence emissions of the induced porphyrins in tissue cells of the surgical margin. In accordance with one example embodiment, the filter in the body may be mechanicaly moved into and out of place in front of the image sensor.

In accordance with a further aspect of the present disclosure, a kit for white light and fluorescence-based visualization of cancerous cells in a surgical margin is disclosed. The kit comprises a handheld, white light and fluorescence-based imaging device for visualizing at least one of precancerous cells, cancerous cells, and satellite lesions in surgical margins and a non-targeted, non-activated compound configured to induce porphyrins in cancerous tissue cells.

In accordance with another aspect of the present disclosure, a multispectral system for visualizing cancerous cells in surgical margins is disclosed. The system comprises a handheld, white light and fluorescence-based imaging device for visualizing at least one of precancerous cells, cancerous cells, and satellite lesions in surgical margins, a display device configured to display data output by the processor of the handheld device; and a wireless real-time data storage and pre-processing device.

In accordance with yet another aspect of the present disclosure, a kit for white light and fluorescence-based visualization of cancerous cells in a surgical margin includes a handheld, white light and fluorescence-based imaging device for visualizing at least one of precancerous cells, cancerous cells, and satellite lesions in surgical margins and a plurality of tips configured to be exchangeable with a tip portion on the handheld device, wherein each tip includes at least one light source.

In accordance with another aspect of the present disclosure, a handheld, white light and fluorescence-based imaging device for visualizing at least one of precancerous cells, cancerous cells, and satellite lesions in surgical margins is disclosed. The device comprises a body having a first end portion configured to be held in a user's hand and a second end portion configured to direct light onto a surgical margin. The body contains at least one excitation light source configured to excite autofluorescence emissions of tissue cells and fluorescence emissions having a wavelength of between about 600 nm and about 660 nm in precancerous cells, cancerous cells, and satellite lesions of the surgical margin after exposure to an imaging or contrast agent. The body also contains a filter configured to prevent passage of reflected excitation light and permit passage of emissions having a wavelength corresponding to autofluorescence emissions of tissue cells and fluorescence emissions between about 600 nm and about 660 nm in tissue cells of the surgical margin. The body further contains an imaging lens, an image sensor configured to detect the filtered autofluorescence emissions of tissue cells and fluorescence emissions between about 600 nm and about 660 nm in tissue cells of the surgical margin, and a processor configured to receive the detected emissions and to output data regarding the detected filtered autofluorescence emissions of tissue cells and fluorescence emissions between about 600 nm and about 660 nm in tissue cells of the surgical margin.

In accordance with a further aspect of the present disclosure, a method of assessing surgical margins is disclosed. The method comprises, subsequent to administration of a compound configured to induce emissions of between about 600 nm and about 660 nm in cancerous tissue cells, positioning a distal end of a handheld, white light and fluorescence-based imaging device adjacent to a surgical margin. The method also includes, with the handheld device, substantially simultaneously exciting and detecting autofluorescence emissions of tissue cells and fluorescence emissions of the induced wavelength in tissue cells of the surgical margin. And, based on a presence or an amount of fluorescence emissions of the induced wavelength detected in the tissue cells of the surgical margin, determining whether the surgical margin is substantially free of at least one of precancerous cells, cancerous cells, and satellite lesions.

In accordance with yet another aspect of the present disclosure, a method of assessing surgical margins is disclosed. The method comprises, subsequent to the administration to a patient of a non-activated, non-targeted compound configured to induce porphyrins in cancerous tissue cells, and with a white light and fluorescence-based imaging device for visualizing at least one of precancerous cells, cancerous cells, and satellite lesions in surgical margins, illuminating tissue cells of a surgical margin in the patient with an excitation light. The method further includes detecting fluorescence emissions from tissue cells in the surgical margin that contain induced porphyrins and displaying in real-time the tissue cells from which fluorescence emissions were detected to guide surgical assessment and/or treatment of the surgical margin.
In accordance with yet another aspect of the present disclosure, a method of assessing lymph nodes is disclosed. The method comprises, subsequent to administration of a compound configured to induce porphyrins in cancerous tissue cells, substantially simultaneously exciting and detecting fluorescence of the induced porphyrins in tissue cells of a target lymph node. The method further includes based on an amount of fluorescence of the induced porphyrins detected in the tissue cells of the target lymph node, determining whether the lymph node is substantially free of cancerous cells.

in accordance with yet another aspect of the present disclosure, a method of predicting an amount of fibrosis in a tissue sample is disclosed. The method comprises receiving RGB data of fluorescence of the tissue sample responsive to illumination with excitation light; and based on a presence or an amount of fluorescence emitted by the tissue sample, calculating a percentage of green fluorescence, a density of the green fluorescence, and a mean green channel intensity of the green fluorescence in the tissue sample.

### BRIEF DESCRIPTION OF THE DRAWINGS

The present disclosure can be understood from the following detailed description either alone or together with the accompanying drawings. The drawings are included to provide a further understanding, and are incorporated in and constitute a part of this specification. The drawings illustrate one or more exemplary embodiments of the present disclosure and together with the description serve to explain various principles and operations,
FIG. 1A is an illustration of the conversion of ALA to PplX in a tumor cell;
FIG. 1B shows peak absorption and emission for PplX;
FIG. 2A is a chart showing exemplary bands of an mCherry filter configured to detect emissions excited by 405 nm excitation light and incorporated into an exemplary embodiment of the handheld multispectral device in accordance with the present disclosure;
FIG. 2B is a cross-sectional view of an exemplary surgical cavity exposed to 405 nm excitation light;
FIG. 3A is a chart showing exemplary bands of an mCherry filter configured to detect emissions excited by 405 nm excitation light and 572 nm excitation light and incorporated into an exemplary embodiment of the handheld multispectral device in accordance with the present disclosure;
FIG. 3B is a cross-sectional view of an exemplary surgical cavity exposed to 405 nm excitation light and 572 nm excitation light, and shows the varying depths of penetration of the different wavelengths of excitation light in accordance with the present teachings;
FIG. 4A is a chart showing exemplary bands of an mCherry filter configured to detect emissions excited by 760 nm excitation light, as well as the absorption and emission wavelengths of the IRdye 800, and incorporated into an exemplary embodiment of the handheld multispectral device in accordance with the present disclosure;
FIG. 4B is a chart showing the absorption and emission wavelengths of the IRdye 800, as well as an exemplary band of a long pass filter configured to detect emissions excited by 760 nm excitation light and incorporated into an exemplary embodiment of the handheld multispectral device in accordance with the present disclosure;
FIGS. 5A-5C show a side view, a perspective view, and an enlarged tip view, respectively, of a first embodiment of a handheld multispectral imaging device in accordance with the present teachings;
FIGS. 5D and 5E show alternative embodiments of a tip for use with the device of FIGS. 5A and 5B;
FIGS. 6A and 6B show a cross-sectional view of the body and a perspective view of the tip of the device of FIGS. 5A-5C;
FIGS. 7A and 7B show a cross-sectional view of a body and a cross-sectional view of a removable tip of a second embodiment of a handheld multispectral imaging device in accordance with the present teachings;
FIGS. 8A and 8B show a cross-sectional view of a body and a cross-sectional view of a tip of a third embodiment of a handheld multispectral imaging device in accordance with the present teachings;
FIGS. 9A and 9B show a cross-sectional view of a body and a cross-sectional view of a removable tip of a fourth embodiment of a handheld multispectral imaging device in accordance with the present teachings;
FIG. 10 is a cross section of a fifth embodiment of a handheld multispectral imaging device in accordance with the present teachings;
FIG. 11 is a cross section of a sixth embodiment of a handheld multispectral imaging device in accordance with the present teachings;
FIGS. 12A and 12B are perspective views of a wireless hub to be used with a handheld multispectral imaging device in accordance with the present teachings;
FIG. 13 is a perspective view of a system for intraoperative visualization of tumor and surgical margins in accordance with the present teachings;
FIG. 14 is a perspective view of a sterilization system for use with a handheld multispectral imaging device in accordance with the present teachings;
FIG. 15 shows a series of photograph images and graphs illustrating a normal tissue autofluorescence profile;
FIG. 16 shows a series of photograph images and graphs illustrating 5-ALA fluorescence in representative invasive breast carcinoma lumpectomy/mastectomy specimens;
FIG. 17 shows WL and FL images of nodes removed during breast cancer surgery;
FIG. 18 shows WL and FL images of mastectomy specimens;
FIG. 19 is a fluorescent image of breast tissue taken during the ALA breast study showing breast tissue comprising 5% fibrosis;
FIG. 20 is a fluorescent image of breast tissue taken during the ALA breast study showing breast tissue comprising 40% fibrosis;
FIG, 21 is a fluorescent image of breast tissue taken during the ALA breast study showing breast tissue comprising 80% fibrosis;
FIG. 22 is a flow chart depicting a method for quantifying the green fluorescence in an image and correlating the amount of green fluorescence in an image to a percentage of fibrosis in a lumpectomy specimen;
FIG. 23 is a flow chart depicting a method of determining the relative composition of a formalin fixed tissue sample stained with H & E;
FIG. 24 is a flow chart depicting a method of determining tumor-to-normal tissue FL color contrast;and
FIG. 25 is a chromaticity diagram for a control group, a low dose group, and a high dose group.

### DESCRIPTION OF VARIOUS EXEMPLARY EMBODIMENTS

Existing margin assessment technologies focus on the excised sample to determine whether surgical margins include residual cancer cells. These technologies are limited by their inability to accurately spatially co-localize a positive margin detected on the excised sample to the surgical bed, a limitation the present disclosure overcomes by directly imaging the surgical cavity.

Other non-targeted techniques for reducing re-excisions include studies which combine untargeted margin shaving with standard of care BCS. While this technique may reduce the overall number of re-excisions, the approach includes several potential drawbacks. For example, larger resections are associated with poorer cosmetic outcomes and the untargeted removal of additional tissues is contradictory to the intention of BCS. In addition, the end result of using such a technique appears to be in conflict with the recently updated ASTRO/SSO guidelines, which defined positive margins as 'tumor at ink' and found no additional benefit of wider margins. Moran MS, Schnitt SJ, Giuliano AE, Harris JR, Khan SA, Horton J et al., "Society of Surgical Oncology-American Society for Radiation Oncology consensus guideline on margins for breast-conserving surgery with whole-breast irradiation in stages I and II invasive breast cancer," Ann Surg Oncol. 2014. 21(3):704-716. A recent retrospective study found no significant difference in re-excisions following cavity shaving relative to standard BCS. Pata G, Bartoli M, Bianchi A, Pasini M, Roncali S, Ragni F., "Additional Cavity Shaving at the Time of Breast-Conserving Surgery Enhances Accuracy of Margin Status Examination," Ann Surg Oncol. 2016. 23(9):2802-2808. Should margin shaving ultimately be found effective, FL-guided surgery may be used to refine the process by adding the ability to target specific areas in a surgical margin for shaving, thus turning an untargeted approach, which indiscriminately removes additional tissue, into a targeted approach that is more in line with the intent of BCS.

The present application discloses devices, systems, and methods for fluorescent-based visualization of tumors, including *in vivo andex* vivovisualization and/or assessment of tumors, multifocal disease, and surgical margins, and intraoperative guidance for removal of residual tumor, satellite lesions, precancerous cells, and/or cancer cells in surgical margins. In certain embodiments, the devices disclosed herein are handheld and are configured to be at least partially positioned within a surgical cavity. In other embodiments, the devices are portable, without wired connections. However, it is within the scope of the present disclosure that the devices may be larger than a handheld device, and instead may include a handheld component. In such embodiments, it is contemplated that the handheld component may be connected to a larger device housing or system by a wired connection.

Also disclosed are methods for intraoperative, *in-vivo* imaging using the device and/or system. The imaging device may be multispectral. It is also contemplated that the device may be hyperspectral. In addition to providing information regarding the type of cells contained within a surgical margin, the disclosed devices and systems also provide information regarding location (i.e., anatomical context) of cells contained within a surgical margin. In addition, methods of providing guidance for intraoperative treatment of surgical margins using the device are disclosed, for example, fluorescence-based image guidance of resection of a surgical margin. The devices, systems, and methods disclosed herein may be used on subjects that include humans and animals.

In accordance with one aspect of the present disclosure, some disclosed methods combine use of the disclosed devices and/or systems with administration of a non-activated, non-targeted compound configured to induce porphyrin in tumor/cancer cells, precancer cells, and/or satellite lesions. For example, the subject may be given a diagnostic dose (i.e., not a therapeutic dose) of a compound (imaging/contrast agent) such as the pro-drug aminolevulinic acid (ALA). As understood by those of ordinary skill in the art, dosages of ALA less than 60 mg/kg are generally considered diagnostic while dosages greater than 60 mg/kg are generally considered therapeutic. As disclosed herein, the diagnostic dosage of ALA may be greater than 0 mg/kg and less than 60 kg/mg, between about 10 mg/kg and about 50 mg/kg, between about 20 mg/kg and 40 mg/kg, and may be administered to the subject in a dosage of about 5 mg/kg, about 10 mg/kg, about 15 kg/mg, about 20 mg/kg, about 25 mg/kg, about 30 mg/kg, about 35 mg/kg, about 40 mg/kg, about 45 mg/kg, about 50 mg/kg, or about 55 mg/kg. The ALA may be administered orally, intravenously, via aerosol, via immersion, via lavage, and/or topically. Although a diagnostic dosage is contemplated for visualization of the residual cancer cells, precancer cells, and satellite lesions, it is within the scope of the present disclosure to use the disclosed devices, systems, and methods to provide guidance during treatment and/or removal of these cells and/or lesions. In such a case, the surgeon's preferred method of treatment may vary based on the preferences of the individual surgeon. Such treatments may include, for example, photodynamic therapy (PDT). In cases where PDT or other light-based therapies are contemplated as a possibility, administration of a higher dosage of ALA, i.e., a therapeutic dosage rather than a diagnostic dosage, may be desirable. In these cases, the subject may be prescribed a dosage of ALA higher than about 60 mg/kg.

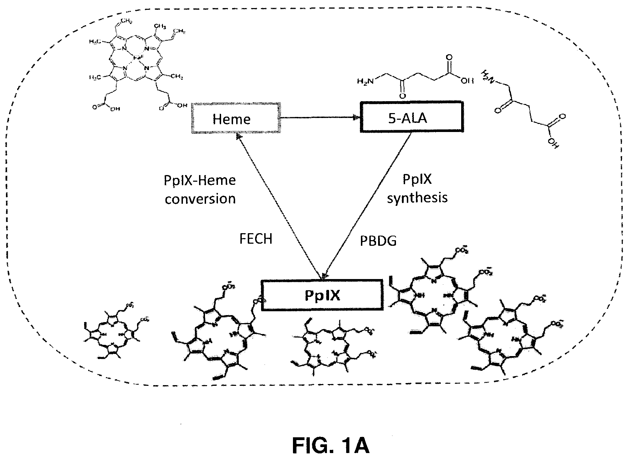

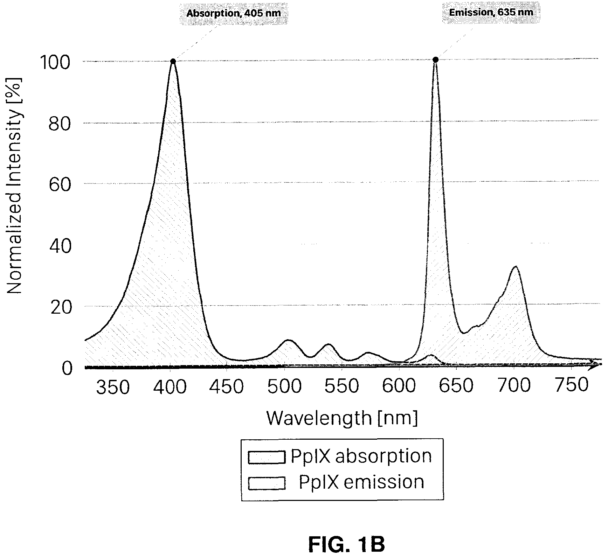

The ALA induces porphyrin formation (protoporphyrin IX (PpIX)) in tumor/cancer cells (FIG. 1A shows the conversion of ALA to PplX within a tumor cell), which when excited by the appropriate excitation light, results in a red fluorescence emission from cells containing the PpIX, which enhances the red-to-green fluorescence contrast between the tumor/cancer tissue cells and normal tissue cells (e.g., collagen) imaged with the device. ALA is non-fluorescent by itself, but PplX is fluorescent at about 630 nm, about 680 nm, and about 710 nm, with the 630 nm emission being the strongest. FIG. 1B illustrates the fluorescence emission of PpIX when excited with excitation light having a wavelength of 405 nm. Alternatively, the endogenous fluorescent difference between tumor/cancer cells or precancer cells and normal/healthy cells may be used without an imaging/contrast agent.

In exemplary embodiments, the non-activated, non-targeted compound configured to induce porphyrin in tumor/cancer cells, precancer cells, and/or satellite lesions is administered to a subject between about 15 minutes and about 6 hours before surgery, about 1 hour and about 5 hours before surgery, between about 2 hours and about 4 hours before surgery, or between about 2.5 hours and about 3.5 hours before surgery. These exemplary time frames allow sufficient time for the ALA to be converted to porphyrins in tumor/cancer cells, precancer cells, and/or satellite lesions. The ALA or other suitable compound may be administered orally, intravenously, via aerosol, via immersion, via lavage, and/or topically.

In cases where the administration of the compound is outside of the desired or preferred time frame, it is possible that PplX may be further induced (or induced for the first time if the compound was not administered prior to surgery) by, for example, applying the compound via an aerosol composition, i.e., spraying it into the surgical cavity or onto the excised tissue (before or after sectioning for examination). Additionally or alternatively, the compound may be administered in a liquid form, for example as a lavage of the surgical cavity. Additionally or alternatively, with respect to the removed specimen, PpIX may be induced in the excised specimen if it is immersed in the liquid compound, such as liquid ALA, almost immediately after excision. The sooner the excised tissue is immersed, the better the chance that PplX or additional PpIX will be induced in the excised tissue.

During surgery, the tumor is removed by the surgeon, if possible. The handheld, white light and fluorescence-based imaging device is then used to identify, locate, and guide treatment of any residual cancer cells, precancer cells, and/or satellite lesions in the surgical bed from which the tumor has been removed. The device may also be used to examine the excised tumor/tissue specimen to determine if any tumor/cancer cells and/or precancer cells are present on the outer margin of the excised specimen. The presence of such cells may indicate a positive margin, to be considered by the surgeon in determining whether further resection of the surgical bed is to be performed. The location of any tumor/cancer cells identified on the outer margin of the excised specimen can be used to identify a corresponding location on the surgical bed, which may be targeted for further resection and/or treatment. This may be particularly useful in situations in which visualization of the surgical bed itself does not identify any residual tumor/cancer cells, precancer cells, or satellite lesions.

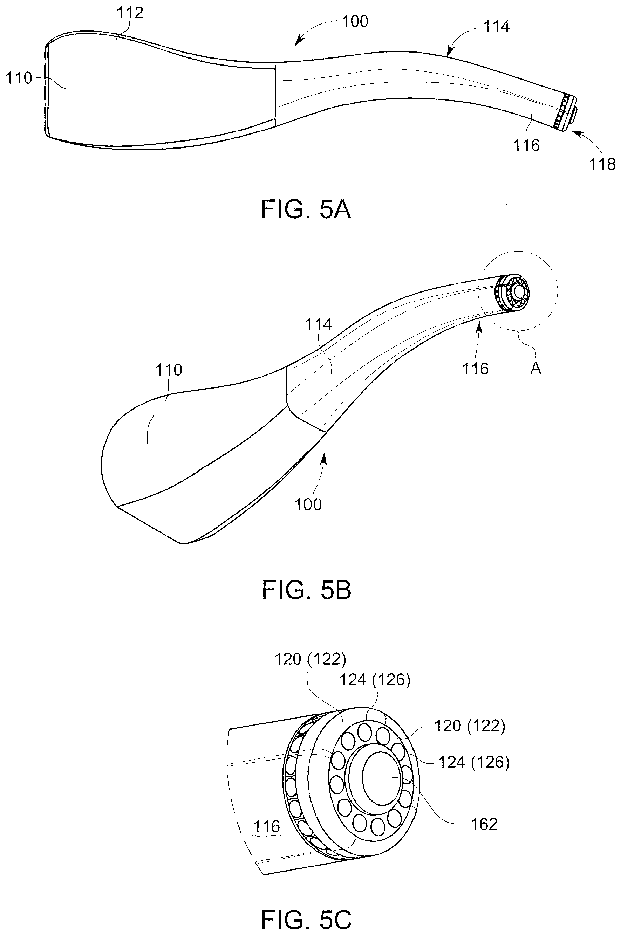

In accordance with one aspect of the present disclosure, a handheld, white light and fluorescence-based imaging device for visualization of tumor/cancer cells is provided. The white light and fluorescence-based imaging device may include a body sized and shaped to be held in and manipulated by a single hand of a user. An exemplary embodiment of the handheld white light and fluorescence-based imaging device is shown in FIGS. 5A-5C. As shown, in some example embodiments, the body may have a generally elongated shape and include a first end portion configured to be held in a user's hand and a second end portion configured to direct light onto a surgical margin on an outer surface of an excised tumor, on one or more sections of the excised tumor, in a surgical cavity from which the tumor/tissue has been excised, or on an exposed surgical bed, The second end may be further configured to be positioned in a surgical cavity containing a surgical margin. The body of the device may comprise one or more materials that are suitable for sterilization such that the body of the device can be subject to sterilization, such as in an autoclave. Examples of a suitable material include polypropylene, polysulfone, polyetherimide, polyphenylsulfone, ethylene chlorotrifluoroethylene, ethylene tetrafluoroethylene, fluorinated ethylene propylene, polychlorotrifluoroethylene, polyetheretherketone, perfluoroalkoxy, polysulfone, polyphenylsulfone, and polyetherimide. Those of ordinary skill in the art will be familiar with other suitable materials. Components within the body of the device that may not be capable of withstanding the conditions of an autoclave, such as electronics, may be secured or otherwise contained in a housing for protection, for example a metal or ceramic housing.

The device may be configured to be used with a surgical drape or shield. For example, the inventors have found that image quality improves when ambient and artificial light are reduced in the area of imaging. This may be achieved by reducing or eliminating the ambient and/or artificial light sources in use. Alternatively, a drape or shield may be used to block at least a portion of ambient and/or artificial light from the surgical site where imaging is occurring. In one exemplary embodiment, the shield may be configured to fit over the second end of the device and be moved on the device toward and away from the surgical cavity to vary the amount of ambient and/or artificial light that can enter the surgical cavity. The shield may be cone or umbrella shaped. Alternatively, the device itself may be enclosed in a drape, with a clear sheath portion covering the end of the device configured to illuminate the surgical site with white light and excitation light.

In some embodiments, the device may include provisions to facilitate attachment of a drape to support sterility of the device. For example, the drape may provide a sterile barrier between the non-sterile device contained in the drape and the sterile field of surgery, thereby allowing the non-sterile device, fully contained in the sterile drape, to be used in a sterile environment. The drape may cover the device and may also provide a darkening shield that extends from a distal end of the device and covers the area adjacent the surgical cavity to protect the surgical cavity area from light infiltration from sources of light other than the device.

The drape or shield may comprise a polymer material, such as polyethylene, polyurethane, or other polymer materials. In some embodiments, the drape or shield may be coupled to the device with a retaining device. For example, the device may include one or more grooves that are configured to interact with one or more features on the drape or shield, in order to retain the drape or shield on the device. Additionally or alternatively, the drape or shield may include a retaining ring or band to hold the drape or shield on the device. The retaining ring or band may include a resilient band, a snap ring, or a similar component. In some embodiments, the drape or shield may be suitable for one-time use.

The drape or shield may also include or be coupled with a hard optical window that covers a distal end of the device to ensure accurate transmission of light emitted from the device. The window may include a material such as polymethyl methacrylate (PMMA) or other rigid, optically transparent polymers, glass, silicone, quartz, or other materials.

The drape or shield may not influence or alter the excitation light of the device. The window of the drape or shield may not autofluoresce under 405 nm or IR/NIR excitations. Additionally, the material of the drape or shield may not interfere with wireless signal transfers to or from the device.

Other variations of a drape or shield configured to reduce or remove ambient and/or artificial light may be used as will be understood by those of ordinary skill in the art.

Additionally or alternatively, the handheld white light and fluorescence-based imaging device may include a sensor configured to identify if lighting conditions are satisfactory for imaging, For example, the device may include an ambient light sensor that is configured to indicate when ambient lighting conditions are sufficient to permit fluorescent imaging, as the fluorescence imaging may only be effective in an adequately dark environment. The ambient light sensor may provide feedback to the clinician on the ambient light level. Additionally, an ambient light level prior to the system going into fluorescent imaging mode can be stored in picture metadata. The light level could be useful during post analysis. The ambient light sensor could also be useful during white light imaging mode to enable the white light LED or control its intensity.

The device may further include, contained within the body of the device, at least one excitation light source configured to excite autofluorescence emissions of tissue cells and fluorescence emissions of induced porphyrins in tissue cells of the surgical margin, surgical bed, or excised tissue specimen. Although use of the device is discussed herein for purposes of examination of surgical margins and/or beds after tissue has been excised and to examine excised tissue specimens, it is contemplated by the inventors and is within the scope of the present application that the devices may be used during excision of the primary tumor, for example as a guide to distinguish between tumor and non-cancerous tissue. Additionally or alternatively, the devices of the present application could also be used to guide removal of satellite lesions and/or tumors. Thus, the device may also be used to make real-time adjustments during a surgical procedure.

As shown in FIGS. 5A-5C, the at least one excitation light source may be positioned on, around, and/or adjacent to one end of the device. Each light source may include, for example, one or more LEDs configured to emit light at the selected wavelength. In some example embodiments, LEDs configured to emit light at the same wavelength may be positioned such that the device emits light in multiple directions. This provides better and more consistent illumination within a surgical cavity.

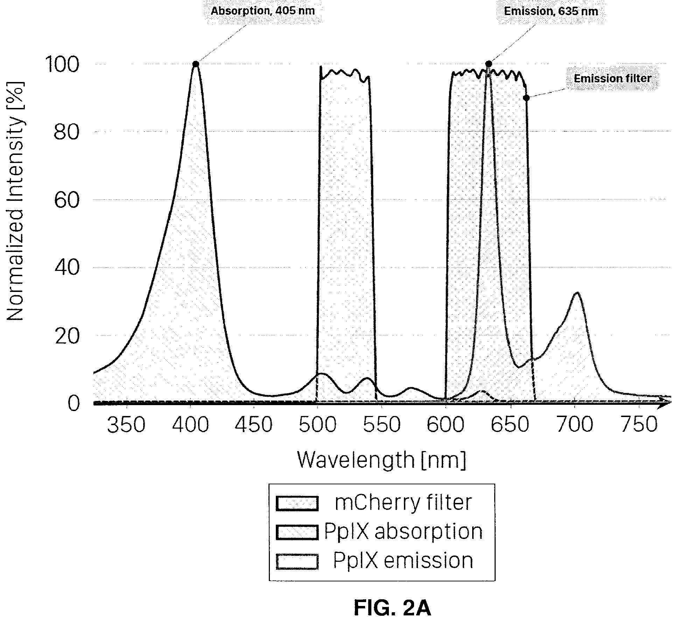

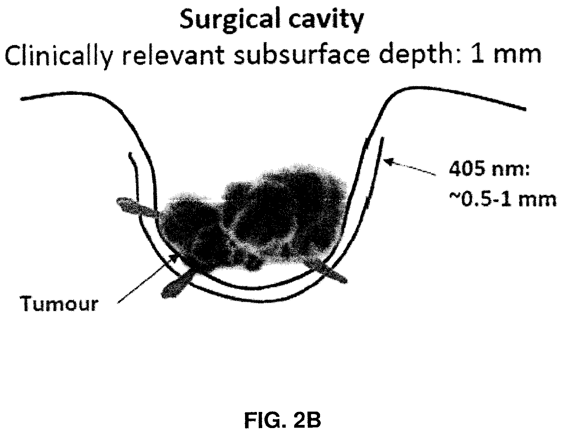

The excitation light source may provide a single wavelength of excitation light, chosen to excite tissue autofluorescence emissions, autofluorescence of other biological components such as fluids, and fluorescence emissions of induced porphyrins in tumor/cancer cells contained in a surgical margin of the excised tumor/tissue and/or in a surgical margin of a surgical bed from which tumor/tissue cells have been excised. In one example, the excitation light may have wavelengths in the range of about 350 nm - about 600 nm, or about 350 nm - about 450 nm and about 550 nm - about 600 nm, or, for example 405 nm, or for example 572 nm. See FIGS. 2A and 2B. The excitation light source may be configured to emit excitation light having a wavelength of about 350 nm - about 400 nm, about 400 nm - about 450 nm, about 450 nm - about 500 nm, about 500 nm - about 550 nm, about 550 nm - about 600 nm, about 600 nm - about 650 nm, about 650 nm - about 700 nm, about 700 nm - about 750 nm, about 750 nm - about 800 nm, about 800 nm - about 850 nm, about 850 nm - about 900 nm, and/or combinations thereof.

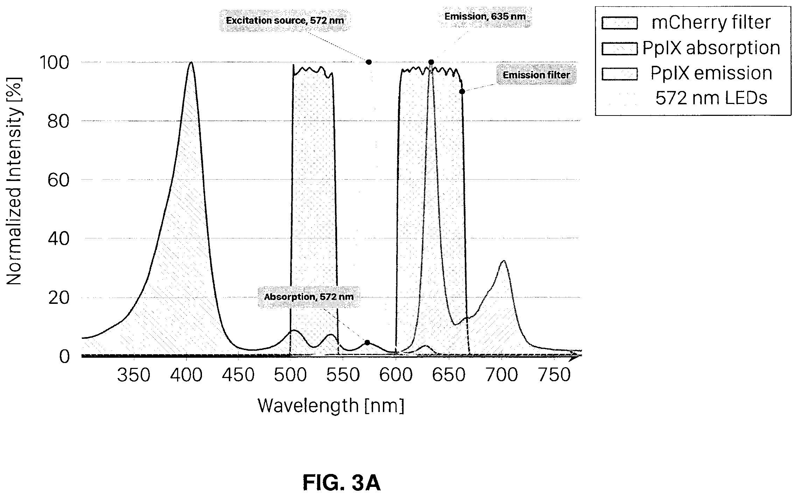

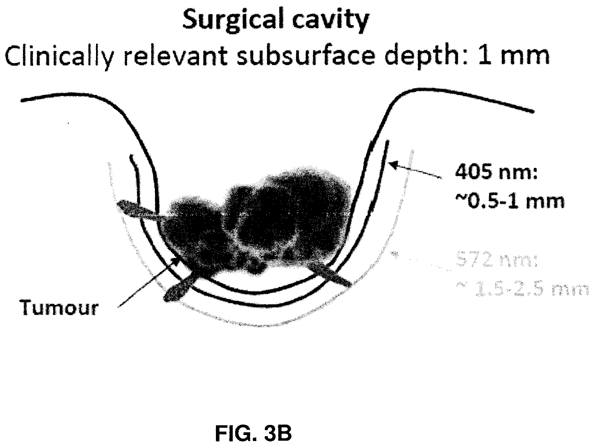

The excitation light source may be configured to provide two or more wavelengths of excitation light. The wavelengths of the excitation light may be chosen for different purposes, as will be understood by those of skill in the art. For example, by varying the wavelength of the excitation light, it is possible to vary the depth to which the excitation light penetrates the surgical bed. As depth of penetration increases with a corresponding increase in wavelength, it is possible to use different wavelengths of light to excite tissue below the surface of the surgical bed/surgical margin. In one example, excitation light having wavelengths in the range of 350 nm - 450 nm, for example about 405 nm ±10 nm, and excitation light having wavelengths in the range of 550 nm to 600 nm, for example about 572 nm ± 10 nm, may penetrate the tissue forming the surgical bed/surgical margin to different depths, for example, about 500µm - about 1 mm and about 2.5 mm, respectively. This will allow the user of the device, for example a surgeon or a pathologist, to visualize tumor/cancer cells at the surface of the surgical bed/surgical margin and the subsurface of the surgical bed/surgical margin. See FIGS. 3A and 3B. Each of the excitation light sources may be configured to emit excitation light having a wavelength of about 350 nm - about 400 nm, about 400 nm - about 450 nm, about 450 nm - about 500 nm, about 500 nm - about 550 nm, about 550 nm - about 600 nm, about 600 nm - about 650 nm, about 650 nm - about 700 nm, about 700 nm - about 750 nm, about 750 nm - about 800 nm, about 800 nm - about 850 nm, about 850 nm - about 900 nm, and/or combinations thereof.

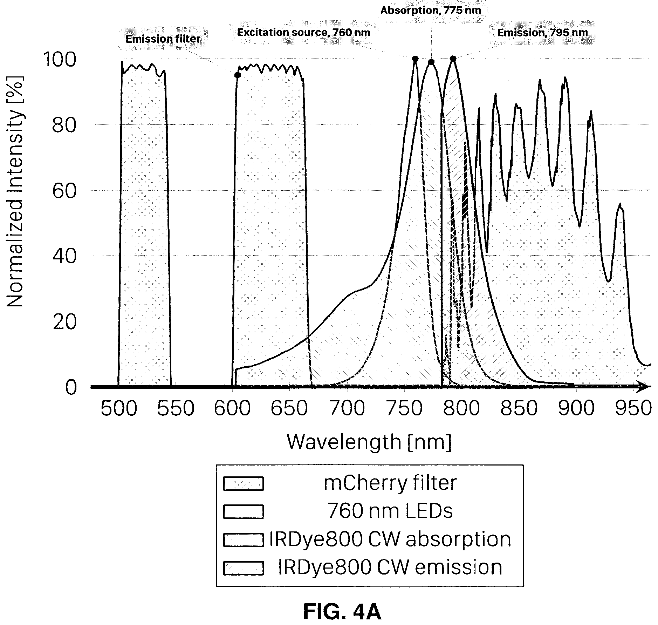

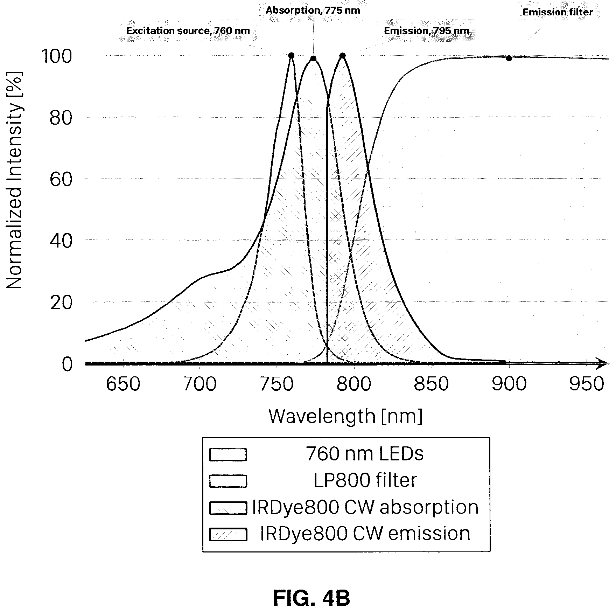

Additionally or alternatively, an excitation light having a wavelength in the near infrared/infrared range may be used, for example, excitation light having a wavelength of between about 760 nm and about 800 nm, for example about 760 nm ± 10 nm or about 780 nm ±10 nm, may be used. In addition, to penetrate the tissue to a deeper level, use of this type of light source may be used in conjunction with a second type of imaging/contrast agent, such as infrared (IR) dye (e.g., IRDye 800, indocyanine green (ICG). See FIGS. 4A and 4B. This will enable, for example, visualization of vascularization, vascular perfusion, and blood pooling within the surgical margins/surgical bed, and this information can be used by the surgeon in making a determination as to the likelihood that residual tumor/cancer cells remain in the surgical bed. In addition, the utility of visualizing vascular perfusion to improve anastomosis during reconstruction would be beneficial,

Thus, excitation light may comprise one or more light sources configured to emit excitation light causing the target tissue containing induced porphyrins to fluoresce, allowing a user of the device, such as a surgeon, to identify the target tissue (e.g., tumor, cancerous cells, satellite lesions, etc.) by the color of its fluorescence. Additional tissue components may fluoresce in response to illumination with the excitation light. In at least some examples, additional tissue components will fluoresce different colors than the target tissue containing the induced porphyrins, allowing the user of the device (e.g., surgeon) to distinguish between the target tissue and other tissues. For example, when excitation light emits light having wavelengths of about 405 nm, the target tissue containing induced porphyrins will fluoresce a bright red color. Connective tissue (e.g., collagen, elastin, etc.) within the same surgical site, margin, bed, or excised specimen, which may surround and/or be adjacent to the target tissue, when illuminated by the same excitation light, will fluoresce a green color. Further, adipose tissue within the same surgical site, margin, bed, or excised specimen, which may surround and/or be adjacent to the target tissue and/or the connective tissue, when illuminated by the same excitation light, will fluoresce a pinkish-brown color. Addition of other wavelengths of excitation light may provide the user (e.g., surgeon) with even more information regarding the surgical site, margin, surgical bed, or excised specimen. For example, addition of an excitation light source configured to emit excitation light at about 572 nm will reveal the above tissues in the same colors, but a depth below the surface of the surgical site, surgical margin, surgical bed, or excised specimen. Alternatively or in addition, the addition of another excitation light, the excitation light being configured to emit excitation light at about 760 nm, will allow the user (e.g., surgeon) to identify areas of vascularization within the surgical site, surgical margin, surgical bed, or surgical specimen. With the use of an NIR dye (e.g., IRDye800 or ICG), the vascularization will appear fluorescent in the near infrared (NIR) wavelength band, in contrast to surrounding tissues that do not contain the NIR dye. For example, the vascularization may appear bright white, grey, or purple in contrast to a dark black background.The device may include additional light sources, such as a white light source for white light (WL) imaging of the surgical margin/surgical bed/tissue specimen/lumpectomy sample, In at least some instances, such as for example, during a BCS such as a lumpectomy, removal of the tumor will create a cavity which contains the surgical bed/surgical margin. WL imaging can be used to obtain an image or video of the interior of the cavity and/or the surgical margin and provide visualization of the cavity. The WL imaging can also be used to obtain images or video of the surgical bed or excised tissue sample. The WL images and/or video provide anatomical and topographical reference points for the user (e.g., surgeon). Under WL imaging, the surgical bed or excised tissues provide useful information to the user (e.g. surgeon and/or pathologist). For example, the WL image can indicate areas of the tissue that contain adipose (fat) tissue, which appear yellow in color, connective tissue, which typically appears white in color, as well as areas of blood, which appear bright red or dark red. Additionally, moisture, charring from cauterization, staining with chromogenic dyes, intraoperative or other exogenous objects (e.g., marking margins, placement of wire guides) can be visualized in the WL images. Furthermore, the WL image may provide context in order to interpret a corresponding FL images. For example, a FL image may provide 'anatomical context' (i.e., background tissue autofluorescence), and the corresponding WL image may allow the user to better understand what is shown in the FL image (e.g., image of a surgical cavity as opposed to an excised specimen). The WL image also . It lets the user colocalize a fluorescent feature in an FL image to the anatomical location under white light illumination.

The white light source may include one or more white light LEDs. Other sources of white light may be used, as appropriate. As will be understood by those of ordinary skill in the art, white light sources should be stable and reliable, and not produce excessive heat during prolonged use.

The body of the device may include controls to permit switching/toggling between white light imaging and fluorescence imaging. The controls may also enable use of various excitation light sources together or separately, in various combinations, and/or sequentially. The controls may cycle through a variety of different light source combinations, may sequentially control the light sources, may strobe the light sources or otherwise control timing and duration of light source use. The controls may be automatic, manual, or a combination thereof, as will be understood by those of ordinary skill in the art.

The body of the device may also contain a spectral filter configured to prevent passage of reflected excitation light and permit passage of emissions having wavelengths corresponding to autofluorescence emissions of tissue cells and fluorescence emissions of the induced porphyrins in tissue cells. In one example embodiment, an mCherry filter may be used, which may permit passage of emissions having wavelengths corresponding to red fluorescence emissions (both autofluorescence and induced porphyrin emissions) and green autofluorescence emissions, wherein the red band captures adipose tissue autofluorescence emissions and PplX emissions and the green band captures connective tissue autofluorescence emissions. As shown in FIGS. 2A -2B and 3A-3B, the green band may permit passage of emissions having a wavelength of between about 500 nm to about 550 nm and the red band may permit passage of emissions having a wavelength of between about 600 nm and 660 nm (it is also possible that the red band may extend between about 600 nm and about 725 nm). The mCherry filter may further comprises a band configured to permit passage of emissions responsive to excitation by infrared excitation light, for example, emissions having a wavelength of about 790 nm and above. See FIG. 4A. Alternatively, instead of an mCherry filter, a plurality of filters may be used, wherein each filter is configured to permit passage of one or more bands of emissions. In one example, an 800 nm long pass filter may be used to capture emissions having a wavelength of 800 nm or greater. See FIG. 4B. Additionally or alternatively, a filter wheel may be used. As will be understood by those of skill in the art, the filter can be further customized to permit detection of other tissue components of interest, such as fluids.

The handheld white light and fluorescence-based imaging device also includes an imaging lens and an image sensor. The imaging lens or lens assembly may be configured to focus the filtered autofluorescence emissions and fluorescence emissions on the image sensor. A wide-angle imaging lens or a fish-eye imaging lens are examples of suitable lenses. A wide-angle lens may provide a view of 180 degrees. The lens may also provide optical magnification. A very high resolution (e.g., micrometer level) is desirable for the imaging device, such that it is possible to make distinctions between very small groups of cells. This is desirable to achieve the goal of maximizing the amount of healthy tissue retained during surgery while maximizing the potential for removing substantially all residual cancer cells, precancer cells, satellite lesions. The image sensor is configured to detect the filtered autofluorescence emissions of tissue cells and fluorescence emissions of the induced porphyrins in tissue cells of the surgical margin, and the image sensor may be tuned to accurately represent the spectral color of the porphyrin fluorescence and tissue autofluorescence. The image sensor may have 4K video capability as well as autofocus and optical zoom capabilities. CCD or CMOS imaging sensors may be used, In one example, a CMOS sensor combined with a filter may be used, i.e., a hyperspectral image sensor, such as those sold by Ximea Company. Example filters include a visible light filter (https://www.ximea.com/en/products/hyperspectral-cameras-based-on-usb3-xispec/mg022hg-im-sm4x4-vis) and an IR filter (https://www.ximea.com/en/products/hyperspectral-cameras-based-on-usb3-xispec/mq022hg-im-sm5x5-nir). The handheld device also may contain a processor configured to receive the detected emissions and to output data regarding the detected filtered autofluorescence emissions of tissue cells and fluorescence emissions of the induced porphyrins in tissue cells of the surgical margin. The processor may have the ability to run simultaneous programs seamlessly (including but not limited to, wireless signal monitoring, battery monitoring and control, temperature monitoring, image acceptance/compression, and button press monitoring). The processor interfaces with internal storage, buttons, optics, and the wireless module. The processor also has the ability to read analog signals.

The device may also include a wireless module and be configured for completely wireless operation. It may utilize a high throughput wireless signal and have the ability to transmit high definition video with minimal latency. The device may be both Wi-Fi and Bluetooth enabled - Wi-Fi for data transmission, Bluetooth for quick connection. The device may utilize a 5 GHz wireless transmission band operation for isolation from other devices, Further, the device may be capable of running as a soft access point, which eliminates the need for a connection to the internet and keeps the device and module connected in isolation from other devices which is relevant to patient data security.

The device may be configured for wireless charging and include inductive charging coils. Additionally or alternatively, the device may include a port configured to receive a charging connection.

In accordance with one aspect of the present disclosure, an example embodiment of a handheld, multispectral imaging device 100, in accordance with the present teachings, is shown in FIGS. 5A-5C. Device 100 includes a body 110 having a first end portion 112 and a second end portion 114. The first end portion 112 is sized and shaped to be held in a single hand by a user of the device. Although not illustrated, the first end portion may include controls configured to actuate the device, toggle between and/or otherwise control different light sources, and manipulate the second end portion 114, when the second end portion is embodied as an articulatable structure.

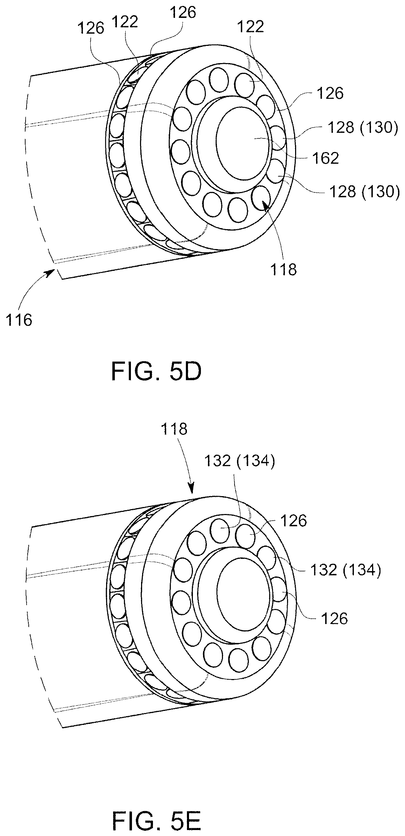

As illustrated in FIGS. 5A-5C, the second end portion 114 of the device 100 may be tapered and/or elongated to facilitate insertion of an end or tip 116 of the second end portion through a surgical incision of 2- 3 cm in size and into a surgical cavity from which a tumor or cancerous tissue has been removed. The end or tip 116 includes light sources around a perimeter or circumference of the end and/or on an end face 118 of the device 100. End face 118 includes, for example, a wide angle lens 162. In one exemplary embodiment, a first white light source 120 comprising white light LEDs 122 is positioned on the tip 116 and end face 118 of the device. A second light source 124 comprising, for example, a 405 nm excitation light source in the form of LEDs 126 is also positioned on the tip 116 and end face 118 of the device 100. In some embodiments, the LEDs 122 and 126 may be arranged in an alternating pattern. In another exemplary embodiment, shown in FIG. 5D, a third light source 128 comprising, for example, a 575 nm excitation light source in the form of LEDs 130 is also positioned on the tip 116 and end face 118. In yet another alternative embodiment, shown in FIG. 5E, a fourth light source in the form of an infrared light source 132 comprising LEDs 134 configured to emit 760 nm excitation light is positioned on the tip 116 and end face 118 of the device 100. As will be understood by those of ordinary skill in the art, these various light sources may be provided in varying combinations, and not all light sources need be provided. In one exemplary embodiment, the tip portion 116 of the device is detachable and is configured to be exchangeable with other tips. In such an embodiment, the tips shown in FIGS. 5C-5E may constitute different tips that are exchangeable on a single device. Additional tips comprising other combinations of light sources and filters are also contemplated by this disclosure. Exemplary tips may include the following combinations of light sources and filters: 405 nm light and mCherry filter; white light without filter; IR/NIR light and 800 nm longpass filter; IR/NIR light and mCherry filter; 405 nm light, IR/NIR light and mCherry filter; 572 nm light and mCherry filter; 405 nm light, 572 nm light and mCherry filter; 405 nm light, 572 nm light, IR/NIR light and mCherry filter; and 572 nm light, IR/NIR light and mCherry filter. Use of exchangeable tips eliminates the design challenge of having to toggle between filters. Other combinations may be created based on the present disclosure, as will be understood by those of ordinary skill in the art.

In embodiments of the device 100 in which the tip 116 is removable and exchangeable, it is envisioned that kits containing replacement tips could be sold. Such kits may be provided in combination with the device itself, or may include one or more compounds or dyes to be used with the types of light sources included on the tips contained in the kit. For example, a kit with a 405 nm light source tip might include ALA, while a kit with a 405 nm light source and a 760 nm light source tip might include both ALA and IRdye 800 and/or ICG. Other combinations of light sources and compounds will be apparent to those of ordinary skill in the art.

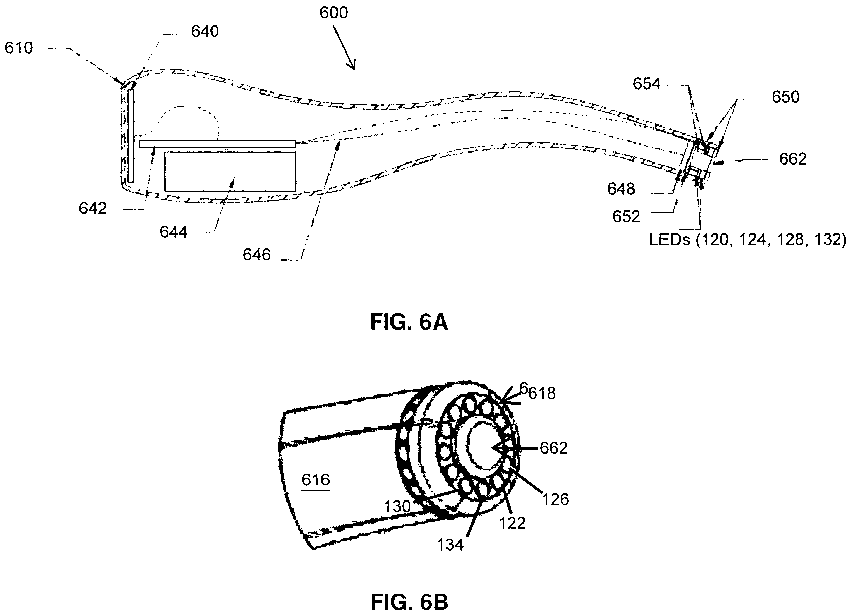

FIGS. 6A and 6B show a cross-sectional view of the device of the embodiment of FIGS. 5A-5C as well as a tip 616 and end face 618 of the device 600. End face 618 includes, for example, a wide angle lens 662. As shown in FIG. 6A, the device 600 includes the device body or housing 610 which contains inductive charging coils 640, an electronics board 642, a battery 644 for powering the various light sources and electronics board, electrical connection(s) 646 for connecting the electronics board 642 to a camera module/image sensor 648 and any of the light sources 120, 124, 128, and 132 which may be present in the tip attached to the body of the device. The light sources are covered by an optically clear window 650. Heat sinks 654 are also provided for the light sources. Positioned in front of the camera module/image sensor 648 is a spectral filter/imaging filter 652. The filter 652 may be mechanically or manually moveable.

In some embodiments, the device may include a polarized filter. The polarizing feature may be part of the spectral filter or a separater filter incorporated inot the spectral filter. The spectral filter/imaging filter may be a polarized filter, for example, a linear or circular polarized filter combined with optical wave plates. This may prevent imaging of tissue with minimized specular reflections (e.g., glare from white light imaging) as well as enable imaging of fluorescence polarization and/or anisotropy-dependent changes in connective tissue (e.g., collagen and elastin). Additionally, the polarized filter may allow a user to better visualize the contrast between different fluorescent colors, and thus better visualize the boundary between different tissue components (e.g. connective vs adipose vs tumor), Stated another way, the polarizing filter may be used for better boundary definition under FL imaging. The polarized filter may also improve image contrast between the tissue components for WL and FL images.

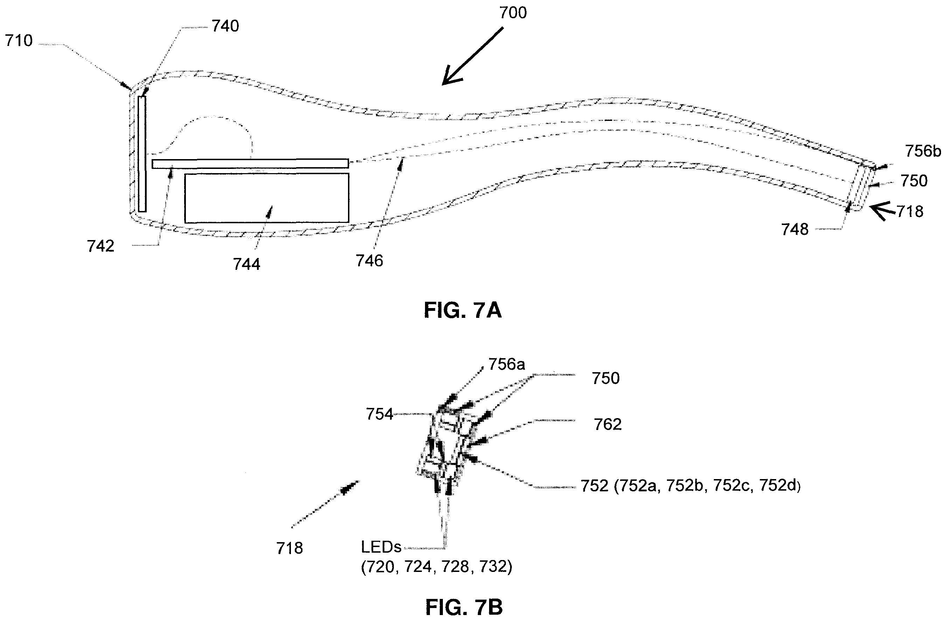

FIGS. 7A and 7B show a cross-sectional view of the body of a second, alternative embodiment of the device and its tip portion, device 700 and end face 718. End face 718 includes, for example, a wide angle lens 762 As shown in FIG. 7A, the device 700 includes the device body or housing 710 which contains inductive charging coils 740 or a charging port (not shown), an electronics board 742, a battery 744 for powering the various light sources, electrical connection(s) 746 for connecting the electronics board 742 to a camera module/image sensor 748 and any of the light sources 120, 124, 128, and 132 which may be present in the tip attached to the body of the device. The light sources are covered by one or more optically clear windows 750. Positioned in front of the camera module/image sensor 748 is a removable spectral filter/imaging filter 752 which forms part of removable tip portion such that it is exchangeable with the tips 116 described above. Each tip includes a separate light source 720, 724, 728, and 732 and the associated filter 752a, 752b, 752c, 752d is configured to prevent passage of reflected excitation light (based on the light source contained on the tip), and to permit passage of emissions responsive to the particular excitation light wavelengths associated with the specific tip. In addition, a heat sink 754 is provided for each LED in the tip of the body 710. The tip of the body 710 further includes an electrical contact 756a configured to contact a corresponding electrical contact 756b on the body 710 of the device 700. It is also contemplated that in some instances only a single light source is included on each tip, and in such instances the tip may not include a filter.

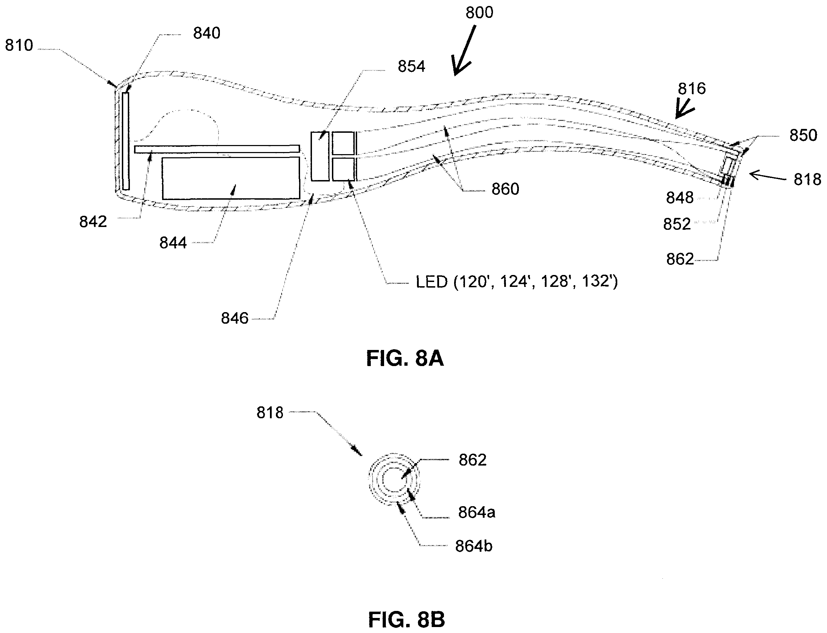

FIGS. 8A and 8B show a cross-sectional view of the body of a third, alternative embodiment of the device and its tip portion, device 800 and end face 818. As shown in FIG. 8A, the device 800 includes the device body or housing 810 which contains inductive charging coils 840 or a charging port (not shown), an electronics board 842, a battery 844 for powering the various light sources, electrical connection(s) 846 for connecting the electronics board 842 to a camera module/image sensor 848. Instead of being provided around a periphery of the housing 810 and/or on an end of the tip of the device 800, light sources are contained within the housing 810 of the device 800. In this embodiment, each light source may utilize a single LED 120', 124', 128', and/or 132'. Each light source is associated with a heat sink. In addition, each light source is associated with a respective light pipe 860 to convey the light from the light source to the end face 818 of the device 800. The tip of the device includes an optically clear window 850, a wide-angle lens 862, an inner light pipe ring 864a, and an outer light pipe ring 864b. The solid light pipe would connect to the ring is as follows: half of the ring (for example, the left half) would be connected to the solid part such that another, smaller light pipe ring could fit concentrically inside. The solid end of this other light pipe, for example, would be connected to the right half of the inner ring. The whole of each ring would project light uniformly, but the light would essentially be delivered to a portion of the ring with adequate diffusion so that the ring emits uniformly. This design could be modified for additional light sources (in this model, each light pipe only transmits light from one source) by adding more concentric rings. Positioned in front of the camera module/image sensor 848 Is a spectral filter/imaging filter 852 which forms part of the tip portion of the body 810. The filter 852 may be mechanically or manually moveable.

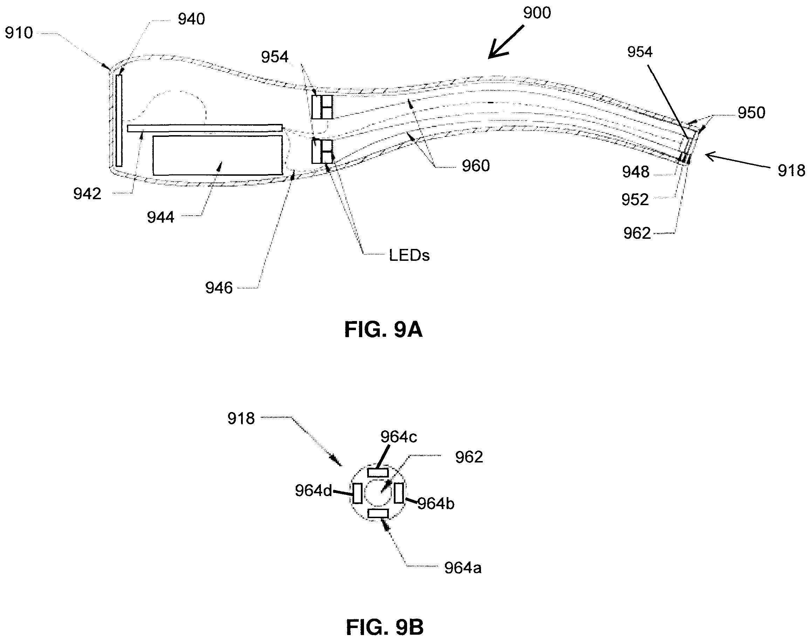

FIGS. 9A and 9B show a cross-sectional view of the body of a fourth, alternative embodiment of the device and its tip portion, device 900 and end face 918. As shown in FIG. 9A, the device 900 includes the device body or housing 910 which contains inductive charging coils 940 or a charging port (not shown), an electronics board 942, a battery 944 for powering the various light sources, electrical connection(s) 946 for connecting the electronics board 942 to a camera module/image sensor 948. Instead of being provided around a periphery of the housing 910 and/or on an end of the tip of the device 900, light sources are contained within the housing 910 of the device 900. In this embodiment, each light source may utilize a multiple LEDs 122, 126, 130, 134. An LED for each light source is positioned adjacent an LED for each other light source present to form a group of LEDs representative of all light sources present, Heat sinks 954are provided for each LED. Each group of LEDs is associated with a respective light pipe 960 to convey the light from the light sources to the tip of the device 900. The tip of the device includes an optically clear window 950, a wide-angle lens 962, and a distal end of each light pipe, e.g., ends 964a, 964b, 964c, and 964d. Positioned in front of the camera module/image sensor 948 is a spectral filter/imaging filter 952 which forms part of the tip portion of the body 910. The filter 652 may be mechanically or manually moveable.

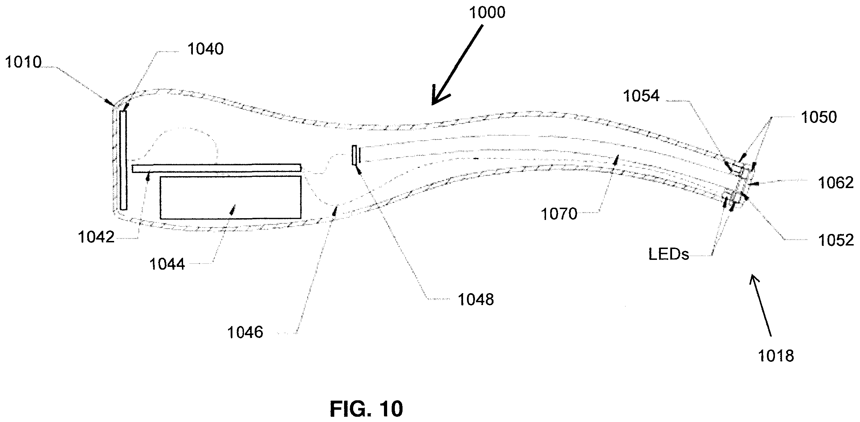

FIG. 10 shows a cross-sectional view of the body of a fifth, alternative embodiment of the device and its tip portion, device 1000 and end face 1018. As shown in FIG. 10, the device 1000 includes the device body or housing 1010 which contains a wide angle lens 1062, inductive charging coils 1040 or a charging port (not shown), an electronics board 1042, a battery 1044 for powering the various light sources, electrical connection(s) 1046 for connecting the electronics board 1042 to a camera module/image sensor 1048 and any of the light sources 120, 124, 128, and 132 which may be present in the tip attached to the body of the device. The light sources are covered by an optically clear window 1050. In addition, a heat sink 1054 is provided for each LED in the tip of the body 1010. In this embodiment, the camera module/image sensor 1048 is spaced away from the tip of the device 1000. Positioned in front of the camera module/image sensor 1048 and between the camera module/image sensor 1048 and a spectral filter/imaging filter 1052 is an image preserving fiber 1070. The image preserving fiber 1070 is used to deliver the emitted light from the distal end pf the device to the camera buried inside where the image is formed. The filter 1052 may be mechanically or manually moveable.

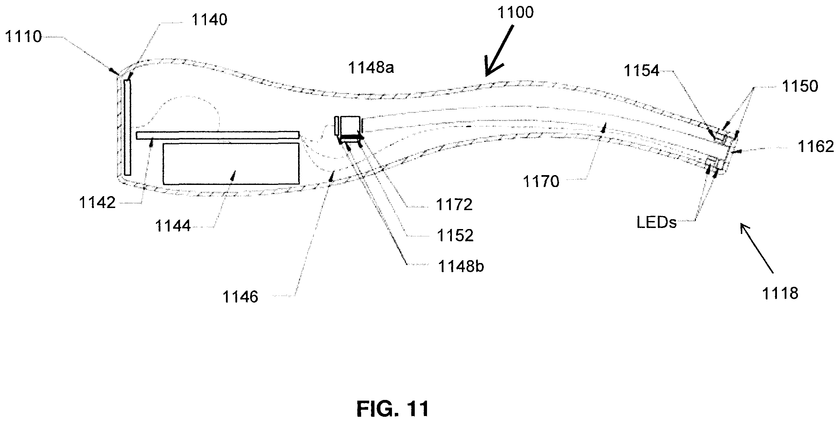

FIG. 1 1 shows a cross-sectional view of the body of a sixth, alternative embodiment of the device and its tip portion, device 1100 and end face 1118. As shown in FIG. 11, the device 1100 includes the device body or housing 1110 which contains inductive charging coils 1140 or a charging port (not shown), an electronics board 1142, a battery 1144 for powering the various light sources, electrical connection(s) 1146 for connecting the electronics board 1142 to two camera module/image sensors 1148a and 1148b as well as any of the light sources 120, 124, 128, and 132 which may be present in the tip on the body of the device. Each light source is associated with a heat sink 1154. The light sources are covered by an optically clear window 1150. Similar to the embodiment of FIG. 10, this sixth embodiment makes use of a light guide/image preserving fiber 1170. As shown, the light guide/image preserving fiber 1170 extends from the wide angle imaging lens 1162 to a beam splitter 1172. On an opposite side of the beam splitter 1172 from the light guide 1170, and directly adjacent to the beam splitter is the first camera module/image sensor 1148a. On a second side of the beam splitter 1170, between the first camera module/image sensor 1148a and the light guide 1170, a spectral filter/imaging filter 1152 is positioned directly adjacent to the beam splitter 1172. Adjacent to the spectral filter/imaging filter 1152 and spaced away from the beam splitter 1172 by the spectral filter/imaging filter 1152, is the second camera modulelimage sensor 1148b. The spectral filter/imaging filter 1152 positioned in front of the second camera module/image sensor 1148b is configured to permit passage of fluorescence emissions responsive to the excitation light sources. The filter 1152 may be mechanically or manually moveable.

This embodiment allows for easy switching between fluorescence (with filter) and white light (no filter) imaging. In addition, both sensors may be capturing images of the exact same field of view at the same time and may be displayed side-by-side on the display. 3D stereoscopic imaging is possible, using both image sensors at the same time, with the filter from the second sensor removed, making it possible to provide a 3D representation of the surgical cavity. In addition, other functions such as Monochrome and full color imaging are possible, with the filter from the second sensor removed. The monochrome and full color images can be combined, with the benefit of a monochrome sensor providing enhanced detail when combined with the full color image.

In each of the embodiments described above, the camera module/image sensor may be associated with camera firmware contained on a processor of the device. The processor is incorporated into the electronics board of the device, as is a wireless module as described above. The camera firmware collects data from the imaging sensor, performs lossless data compression and re-sampling as required, packages image and video data appropriate to the transmission protocol defined by the soft access point, timestamps data packages for synchronization with audio annotation data where applicable, and transmits the data to be received by a wireless hub in real time.

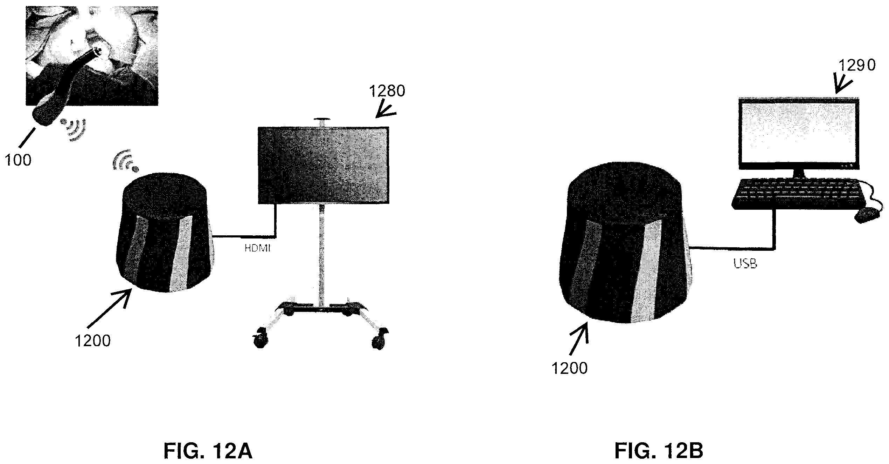

The handheld, multispectral imaging device is configured to be operatively coupled with a wireless hub 1200. As shown in FIG. 11A, the wireless hub 1200 is configured to receive data from the device 100 and transmit the data, via a wired connection, to a display device 1280 positionable for viewing by an operator of the device or others nearby. The wireless hub 1200 includes memory for storing images and audio. The wireless hub may include a microphone for recording audio during use of the device 100 and timestamping the audio for later synchronization with the image/video data transmitted by the device. The wireless hub 1200 includes firmware configured to receive data from the camera module in real time, decompress image and video data, pre-process data (noise removal, smoothing) as required, synchronize audio and video based on timestamp information, and prepare data for wired transmission to the display. It is also contemplated that the hub may be wired to the device and/or form a part of the circuitry of the device when the device itself is not wireless. Additionally, after completion of the surgical procedure in the operating theater, the wireless hub 1200 may be plugged into computer 1290 running cataloguing and analysis software to import images/videos (see FIG. 12B).

The display 1280 may be any display that can be utilized in a surgical suite or in a lab. The display 1280 includes firmware configured to transmit image, video and audio data via a wired connection to an external display monitor, display video data in real time with image capture indication, display images from different light sources side by side up command, and integrate with external augmented reality and virtual reality systems to prepare/adjust display settings as per user preference.

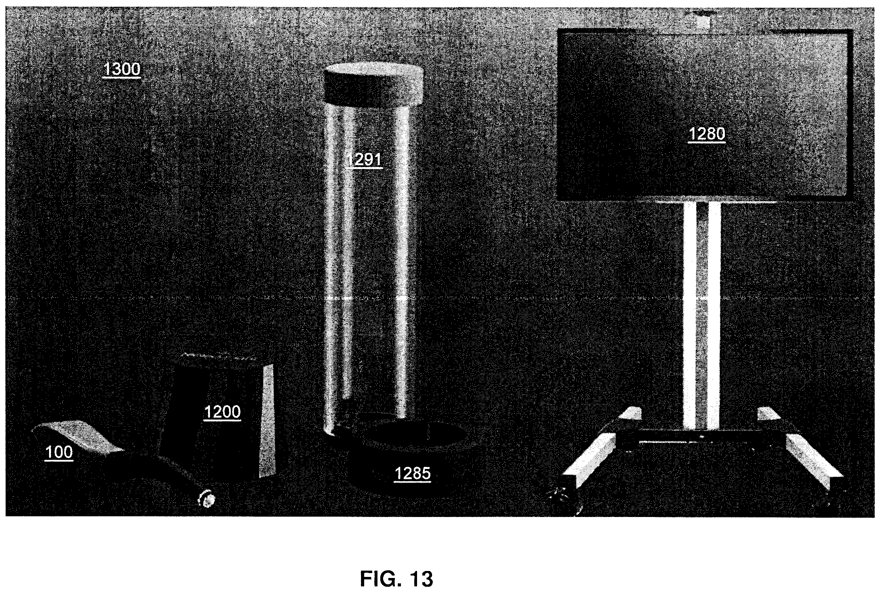

Together, the handheld multispectral imaging device 100, the wireless hub 1200, and the display 1280 form a system 1300 configured to permit intraoperative visualization of tumor and surgical margins. The system may include other components as well. For example, as shown in FIG. 13, a system 1300 configured to permit intraoperative visualization of tumor and surgical margins, may include a handheld multispectral imaging device 100, a wireless hub 1200, a display 1280, a wireless charging dock 1285, and an autoclave container 1291. Although not pictured, the system may further include a non-activated, non-targeted compound configured to induce porphyrins in tumor/cancer tissue cells.

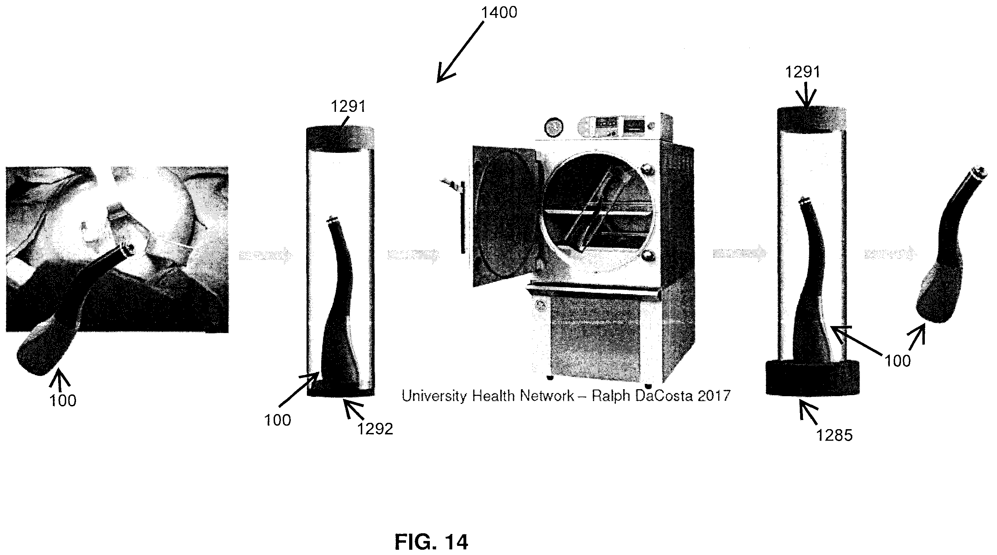

As shown in FIG. 14, an autoclave container 1291 maybe provided as part of a sterilization system for use with device 100. FIG. 14 illustrates a cylindrical autoclave container 1291, although containers of other shapes are contemplated. The container 1291 may have a base 1292 configured to receive and support a base of the device 100. As previously described above, the base of the device 100 may include inductive charging coils for wireless charging. The base 1292 of the container may be configured to fit within the wireless charging dock 1285 and permit wireless charging of the device 100 while keeping the device 100 in sterilized, ready-to-use condition. For example, the container may form a transparent casing so that the device 100 and an indicator strip can be seen without opening the casing and thus compromising sterility. In one example, the device 100 is utilized for imaging, it's surfaces are then sanitized and it is placed in an autoclave case with autoclave indicator strip. The case with device 100 is placed in autoclave and sterilized, the case is then removed from autoclave and sealed, placed on charging dock 1285 where it sits until ready for next surgery. This integrated sterilizing and charging process will ensure compliance with biosafety requirements across global hospital settings.

In accordance with the present teachings, an exemplary method of using the device 100 will now be described. Prior to surgery, the patient is prescribed a diagnostic dosage of a non-activated, non-targeted compound configured to induce porphyrins in tumor/cancer tissue cells, such as ALA. The dosage may comprise, for example, about 5 mg/kg, about 10 mg/kg, about 15 mg/kg, about 20 mg/kg, about 25 mg/kg, about 30 mg/kg, about 35 mg/kg, about 40 mg/kg, about 45 mg/kg, about 50 mg/kg, or about 55 mg/kg. As also discussed above, it is possible to administer a dosage greater than about 60 mg/kg. The patient is provided with instructions to consume the compound between about 15 min and about 6 hours prior to surgery, between about 1 and about 5 hours prior to surgery, or between about 2 and about 4 hours before surgery. If the patient is unable to take the compound orally, it may be administered intravenously. Additionally or alternatively, as previously discussed, it is possible to administer the compound as an aerosol or a lavage during surgery.

The pro-drug aminolevulinic acid (ALA) induces porphyrin formation in tumor/cancer tissue cells via the process illustrated in FIG. 1. An example of an appropriate ALA formulation is commercially available under the name Gliolan (Aminolevulinic acid hydrochloride), made by Photonamic GmbH and Co. This compound is commonly referred to as 5-ALA. Another exemplary source of ALA is Levulan^{®} Kerastick^{®}, made by Dusa Pharmaceuticals Inc. As discussed above, the use of diagnostic dose of ALA or 5-ALA may induce PplX formation in the tumor/cancer tissue cells and hence may increase the red fluorescence emission, which may enhance the red-to-green fluorescence contrast between the tumor/cancer tissue cells and healthy tissue imaged with the device.

In one example, oral 5-ALA was dissolved in water and administered by a study nurse between 2-4 h before surgery in patients at dosages of 15 or 30 mg/kg 5-ALA. The PRODIGI device, used in clinical trials described herein is also described in U.S. Patent No. 9,042,967, entitled "Device and method for wound imaging and monitoring,".

Approximately 2-4 hours after 5-ALA or a similar compound is administered, the surgery begins. In this application, surgical processes are described relative to BCS. However, the scope of the present application is not so limited and is applicable to surgeries and pathological analyses for all types of cancer, including for example, breast cancer, brain cancer, colorectal cancer, squamous cell carcinoma, skin cancer, prostate cancer, melanoma, thyroid cancer, ovarian cancer, cancerous lymph nodes, cervical cancer, lung cancer, pancreatic cancer, head and neck cancer, or esophageal cancer. Additionally, the methods and systems disclosed herein may be used with regard to cancers in animals excluding humans, for example, in canines or felines. The methods and systems may be applicable with, for example, mast cell tumors, melanoma, squamous cell carcinoma, basal cell tumors, tumors of skin glands, hair follicle tumors, epitheliotropic lymohoma, mesenchymal tumors, benign fibroblastic tumors, blood vessel tumors, lipomas, liposarcomas, lymphoid tumors of the skin, sebaceous gland tumors, and soft tissue sarcomas in canines and felines,

The surgeon begins by locating the tumor and subsequently removing the tumor. As discussed above, the surgeon may use the imaging device for location of the tumor, especially in cases where the tumor comprises many tumor nodules. Additionally, the surgeon may also use the imaging device during resection of the tumor to look at margins as excision is taking place (in a manner substantially the same as that described below). After the surgeon removes the tumor/cancerous tissue, the distal end 114 of the device 100, including at least the tip 116 and end face 118 are inserted through the surgical incision into the surgical cavity from which the tumor/cancerous tissue has been removed. The surgeon operates the controls on the proximal portion of the device, held in the surgeon's hand, to actuate the white light source and initiate white light imaging (WL imaging) of the surgical cavity and surgical bed. During WL imaging, the spectral filter is not engaged and light reflected from the surfaces of the surgical cavity passes through the wide-angle imaging lens and is focused on the camera module/image sensor in the body 110 of the device 100. The processor and/or other circuitry on the electronics board transmits the image data (or video data) to the wireless hub 1200, wherein the data is stored and/or pre-processed and transmitted to the display 1280. The surgeon/device operator may move the tip of the device around in the surgical cavity as necessary to image the entire cavity (or as much of the cavity as the surgeon desires to image). In some embodiments, the distal end portion of the device may be articulatable and is controlled to articulate the distal end portion thereby changing the angle and direction of the white light incidence in the cavity as needed to image the entire cavity. Articulation of the distal end portion may be achieved by various means, as will be understood by those of ordinary skill in the art. For example, the distal end may be manually articulatable or it may be articulatable by mechanical, electromechanical, or other means.

Subsequent to WL imaging, the surgeon/device operator, toggles a switch or otherwise uses controls to turn off the white light source and actuate one or more of the excitation light sources on the device 100. The excitation light source(s) may be engaged individually, in groups, or all at once. The excitation light source(s) may be engaged sequentially, in a timed manner, or in accordance with a predetermined pattern. As the excitation light source(s) is actuated, excitation light is directed onto the surgical bed of the surgical cavity, exciting autofluorescence emissions from tissue and fluorescence emissions from induced porphyrins in tumor/cancer tissue cells located in the surgical margin. The imaging lens on the end face 118 of the device 100 focuses the emissions and those emissions that fall within wavelength ranges permitted passage by the spectral filter pass through the filter to be received by the camera module/image sensor within the device body 110. The processor and/or other circuitry on the electronics board transmits the image data (or video data) to the wireless hub 1200, wherein the data is stored and/or pre-processed and transmitted to the display 1280. Thus, the surgeon may observe the captured fluorescence images on the display in real time as the surgical cavity is illuminated with the excitation light. This is possible due to the substantially simultaneous excitation and detection of the fluorescence emissions. As the surgeon observes the fluorescence images, it is possible to command the display of the white light image of the same locality in a side-by-side presentation on the display. In this way, it is possible for the surgeon to gain context as to the location/portion of the surgical cavity/surgical bed or margin being viewed, This allows the surgeon to identify the location of any red fluorescence in the cavity/margin, which may be attributable to residual cancer cells in the cavity/margin. In addition to red fluorescence, the FL imaging may also capture green fluorescence representative of connective tissue such as collagen. In some cases, the autofluorescence emissions forming very dense connective tissue in the breast will fluoresce a bright green color. This allows the surgeon to identify areas of dense connective tissue, differentiate from dark areas which may represent the vasculature/vascularization (dark due to the absorption of light), as a more highly vascularized tissue may potentially represent vascularization associated with cancerous cells. Additionally, by viewing the autofluorescence of the connective tissue in conjunction with any red fluorescence, the surgeon is given context regarding the location of the red fluorescence that may represent residual cancer cells. This context may be used to inform the surgeon's decision regarding further treatment and/or resection of the surgical bed/surgical margin as well as for decisions regarding reconstruction procedures,

As with WL imaging, during FL imaging, the surgeon/device operator may move the tip of the device around in the surgical cavity as necessary to image the entire cavity (or as much of the cavity as the surgeon desires to image). In some embodiments, the distal end portion of the device may be articulatable and is controlled to articulate the distal end portion thereby changing the angle and direction of the white light incidence in the cavity as needed to image the entire cavity. Articulation of the distal end portion may be achieved by various means, as will be understood by those of ordinary skill in the art. For example, the distal end may be manually articulatable or it may be articulatable by mechanical, electromechanical, or other means.

Although this process is described with WL imaging occurring prior to FL imaging, it is possible to reverse the process and/or to perform FL imaging without WL imaging.

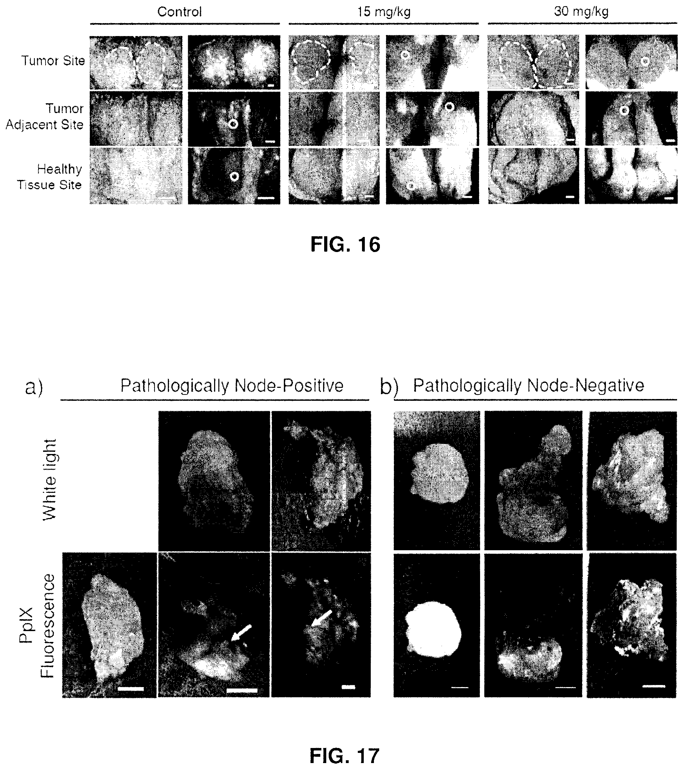

In addition to viewing the surgical margins of a surgical cavity, the disclosed handheld multispectral imaging device may also be used to observe lymph nodes that may be exposed during the surgical procedure. By viewing lymph nodes prior to removal from the subject's body, it is possible to observe, using the device 100, red fluorescence emissions from cells containing induced porphyrins that are within the lymph node. Such an observation is an indication that the tumor/cancer cells have metastasized, indicating that the lymph nodes should be removed and that additional treatment may be necessary. Use of the imaging device in this manner allows the device to act as a staging tool, to verify the stage of the cancer and/or to stage the cancer dependent upon the presence or absence of red fluorescence emissions due to induced porphyrins in the lymph node. Such a process may also be used on lymph nodes that have already been removed from the subject, to determine whether tumor/cancer cells are contained within the removed lymph nodes, Independent of the process used, *in vivo, ex vivo or in vitro,* the information obtained can be used to inform the surgeon's decisions regarding further treatment and/or interventions. FIG. 17 shows WL and FL images of nodes removed during breast cancer surgery. In FIG. 17, WL (top) and FL (bottom) images of excised lymph nodes: a) PpIX FL detected in tumour-positive sentinel nodes from 3 high dose ALA patients (left panel: grossly obvious; centre/right panels: grossly occult); b) Tumour-negative nodes from 5-ALA patients that show characteristic green and pink FL signatures of normal connective and adipose tissue, respectively. Scale *bar = 0.5 cm.*

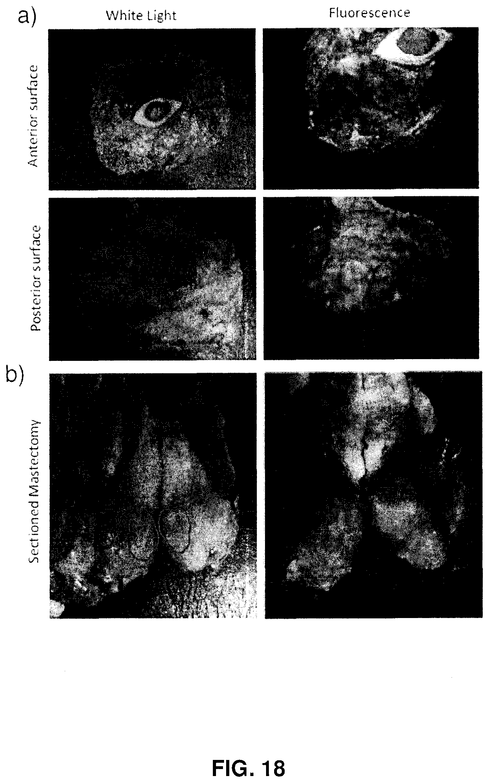

In addition to looking at the surgical cavity and the lymph nodes, there is also value in imaging the removed tumor. The outer surface (surgical margin) of the tumor can be imaged, looking to identify cancer cells, precancer cells, and satellite lesions. The removed tissue can also be viewed with the imaging device after sectioning. FIG. 18 shows WL and FL images of mastectomy specimens removed during breast cancer surgery. In FIG. 18, WL (left) and FL (right) images of (a) intact and (b) serially sectioned mastectomy specimen from a patient administered 30 mg/kg 5-ALA are shown. Blue line demarcates the palpable tumor border.

In accordance with another aspect of the present disclosure, it is contemplated that the intensity of the induced porphyrins detected may be used as a guide to determine an optimal time frame for PDT. For example, it is possible to monitor the intensity of the fluoresce emitted by the porphyrins and determine when they are at peak, and perform PDT at that time for optimal results.

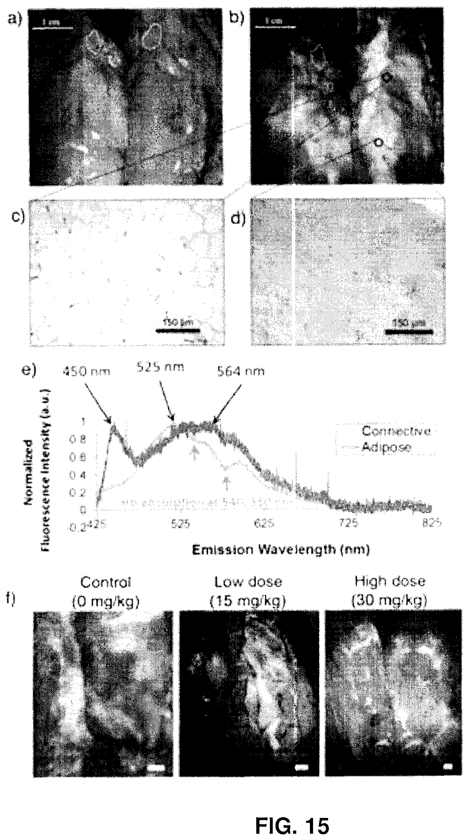

Under standard WL, differentiating between regions of breast adipose and connective tissues is challenging. FL imaging reveals consistent autofluorescent (AF) characteristics of histologically validated adipose and connective tissues, which appear pale pink and bright green, respectively, under 405 nm excitation. When combined with 5-ALA red FL, the differing emission spectra of normal tissue AF and PpIX are easily distinguishable visually (see FIGS. 15 and 16). Collagen and elastin, major components of breast connective tissue, are well known for their AF properties and have been shown to emit in the green (490-530 nm) range of the visible light spectrum when excited at 405 nm. The 405 nm excitation LEDs and the dual band emission filter (500-545 nm and 600-660 nm) are suitable for breast tumor imaging using 5-ALA because they provide a composite image comprised of red PplX and green connective tissue FL, and broad green-to-red FL of adipose tissue (appears pink). While secondary to the primary objective of differentiating cancerous from normal tissue, spatial localization of adipose and connective tissue provides image-guidance with anatomical context during surgical resection of residual cancer, thus sparing healthy tissues to preserve cosmesis,

AF mammary ductoscopy using blue light illumination can spectrally differentiate between healthy duct luminal tissue AF (bright green) and invasive breast tumor tissue. The clinicians' imaging data demonstrates bright green AF in areas of healthy breast tissue. Moreover, the clinical findings with 5-ALA demonstrate that both en face FL imaging and endoscopic FL imaging are clinically feasible.

During one clinical trial, tumor AF intensity and distribution were heterogeneous. Qualitatively, intensity ranged from visually brighter, darker, or low contrast compared to surrounding normal breast tissue. In addition, mottled green FL was common among the specimens both in the demarcated tumor as well as in areas of normal tissue, likely due to interspersed connective tissue. Endogenous tumor AF was inconsistent across different patient resection specimens and hence is not a reliable intrinsic FL biomarker for visual identification of tumors within surgical breast tumor specimens (i.e., not all tumors are brighter compared to surrounding normal tissues).

Overall, differences in tumor AF signals may represent differences in the composition of each tumor and the surrounding normal regions. It is possible that brighter tumors contain more fibrous connective tissue and as a result had a characteristic bright green AF signature. However, in cases where the healthy surrounding tissue was also highly fibrous with dense connective tissue, the tumor and normal AF signal were similar and could not be distinguished from each other, resulting in low contrast of the tumor relative to normal tissue.

Blood is known to increase absorption of 405 nm light resulting in decreased emission. Intact specimens were rinsed with saline prior to imaging to remove surface blood, however, once bread-loafed, blood in tumor vessels may have affected the AF intensity of tumor sections. Therefore, it is possible that darker tumors had lower connective tissue content and higher vascularity.

In patients receiving 5-ALA, PpIX FL was lower in areas of normal connective and adipose tissue relative to tumor tissue. While the diagnostic measures for detecting tumor were not significantly improved in the higher 5-ALA group, the inventors did see an increase in the median concentration of tumor PplX relative to the lower 5-ALA group.

The inventors found connective tissue (collagen) was characterized by green AF (525 nm peak) when excited by 405 nm light. Accordingly, necrotic areas that were also highly fibrotic were characterized by green AF. Additionally, collagen and elastin found in the intimal and adventitial layers of tumor-associated vasculature exhibited bright green AF. Broad AF emission between 500 nm and 600 nm was observed in adipocytes located in both healthy and tumor tissues. This is likely due to the broad emission spectrum of lipo-pigments. Under macroscopic imaging with an alternative embodiment of the imaging device, the broad 500-600 nm FL emission characteristic of adipocytes is spectrally and visually distinct from the narrow red (635 nm peak) FL emission characteristic of tumor-localized of PpIX. Thus, tumor cells containing PpIX are distinguishable from a background of fatty breast tissues.

Multispectral or multiband fluorescence images using 405 nm (e.g., +/- 5 nm) excitation, and detecting ALA-induced porphyrin FL between 600-750 nm, can be used to differentiate between connective tissues, adipose tissues, muscle, bone, blood, nerves, diseased, precancerous and cancerous tissues.

Device and method can be used to visualize microscopic and macroscopic tumor foci (from a collection of cells to mm-sized or larger lesions) at the surface or immediately below the surface of a resected specimen (lumpectomy, mastectomy, lymph node) and/or surgical cavity, and this can lead to:
Better visualization of tumor foci/lesions against a background of healthy or inflamed or bloody tissues;
Faster detection of microscopic tumor foci/lesions using FL imaging compared with conventional methods;
Real-time visual guidance from FL images/video for the clinician to remove the FL tumor foci/lesions during surgery;
Confirmation of more complete tumor removal following FL imaging (reduction of porphyrin FL or its absence after FL guided surgery can indicate more (or all of) the tumor has been removed;
FL images can be used to target biopsy of suspicious premalignant or malignant tissues in real time;
FL imaging can also identify macroscopic and microscopic tumor foci/lesions in lymphatic tissues during surgery, including lymph nodes;
Area of PpIX red fluorescence indicates extent of tumour burden in lymph node;
Detect subsurface tumor lesions during or after a surgical procedure;
Differentiation between low, moderate and high mitotic index tumor lesions based on porphyrin FL intensity and color;
FL images and video with audio annotation to document completeness of tumour removal;
Can be correlated with pathology report and used to plan re-excision, reconstructive surgery;
FL images/video can be used to plan treatment of focal x-ray radiation or implantation of brachytherapy seed treatment in breast or other types of cancer;
Improve margin assessment by detecting microscopic residual tumor foci/lesions; and
Connective tissues FL green, Premalignant and malignant tissues (red).

FL imaging can be used in combination with FL point spectroscopy, Raman spectroscopy and imaging, mass spectrometry measurements, hyperspectral imaging, histopathology, MRI, CT, ultrasound, photoacoustic imaging, terahertz imaging, infrared FL imaging, OCT imaging, polarized light imaging, time-of-flight imaging, bioluminescence imaging, FL microscopy for examining ex vivo tissues and/or the surgical cavity for the purpose of detecting diseased tissue, diagnosing said diseased tissue, confirming the presence of healthy tissues, guiding surgery (or radiation or chemotherapy or cell therapies in the case of patients with cancer).

### PREDICTIVE VALUE AND USE OF IMAGE DATA

In addition to the ability to identify cancer or precancer cells, the image data gathered through use of the devices and methods disclosed herein can be used for several purposes.

### Fibrosis

In accordance with one aspect of the present disclosure, the image data gathered using the devices and methods disclosed herein may be useful in the identification of fibrosis. Fibrosis refers to a thickening or increase in the density of breast connective tissue. Fibrous breast tissues include ligaments, supportive tissues (stroma), and scar tissues. Breast fibrosis is caused by hormonal fluctuations, particularly in levels of estrogen, and can be more acute just before the menstruation cycle begins. Sometimes these fibrous tissues become more prominent than the fatty tissues in an area of the breast, possibly resulting in a firm or rubbery bump. Fibrosis may also develop after breast surgery or radiation therapy. The breast reacts to these events by becoming inflamed, leaking proteins, cleaning up dead breast cells, and laying down extra fibrous tissue. Fibrous tissue becomes thinner with age and fibrocystic changes recede after menopause.

In the fluorescence RGB images collected with the PRODIGI camera in the breast ALA study, connective tissue in the breast appears as green colour fluorescence. This is expected as this reflects the wavelengths emitted by collagen when excited with 405 nm light, and, collagen is the primary component of connective tissue. Therefore, by characterising and quantifying the green autofluorescence in the images, a correlation to the connective tissue fibrosis can be performed.

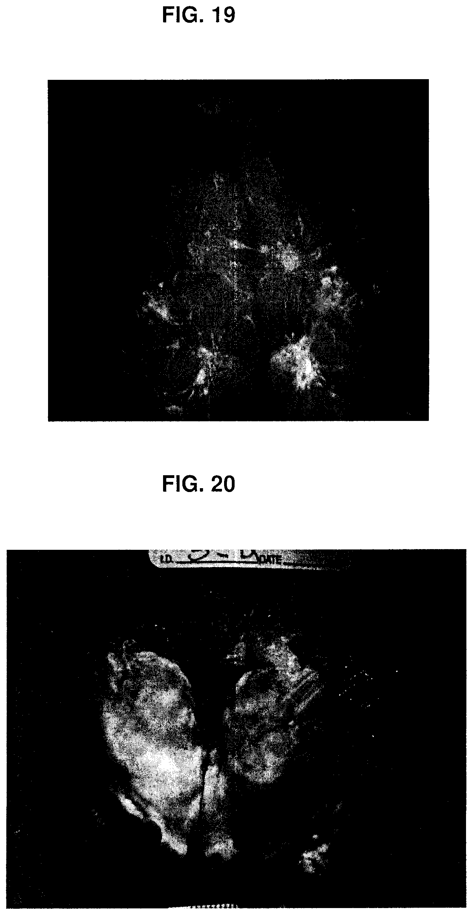

FIG. 19 is a first example image taken during treatment of a patient during the ALA breast study. Clinicians in the study reported an amount of five percent (5%) fibrosis as corresponding to the percentage of fibrosis found in lumpectomy specimen shown in FIG. 19. As can be seen in FIG. 19, the amount of green fluorescence visible approximately correlates to about 5% percent of the tissue in the image.

FIG. 20 is a second example image taken during treatment of a patient during the ALA breast study. Clinicians in the study reported an amount of forty percent (40%) fibrosis as corresponding to the percentage of fibrosis found in lumpectomy specimen shown in FIG. 20. As can be seen in FIG. 20, the amount of green fluorescence visible approximately correlates to about 40% percent of the tissue in the image.

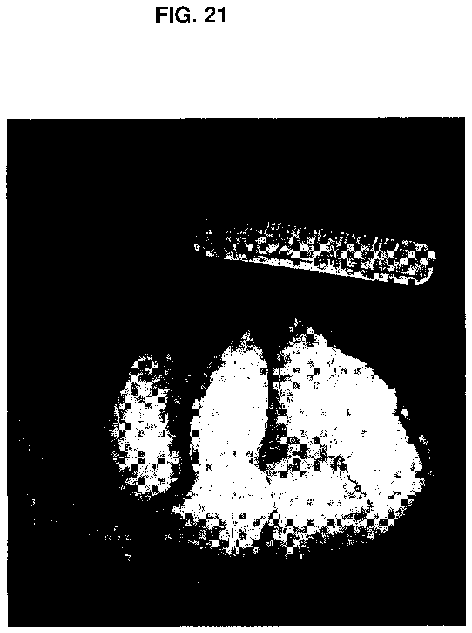

FIG. 21 is a second example image taken during treatment of a patient during the ALA breast study. Clinicians in the study reported an amount of eighty percent (80%) fibrosis as corresponding to the percentage of fibrosis found in lumpectomy specimen shown in FIG. 21. As can be seen in FIG. 21, the amount of green fluorescence visually observable in the fluorescence image approximately correlates to about 80% percent of the tissue in the image.

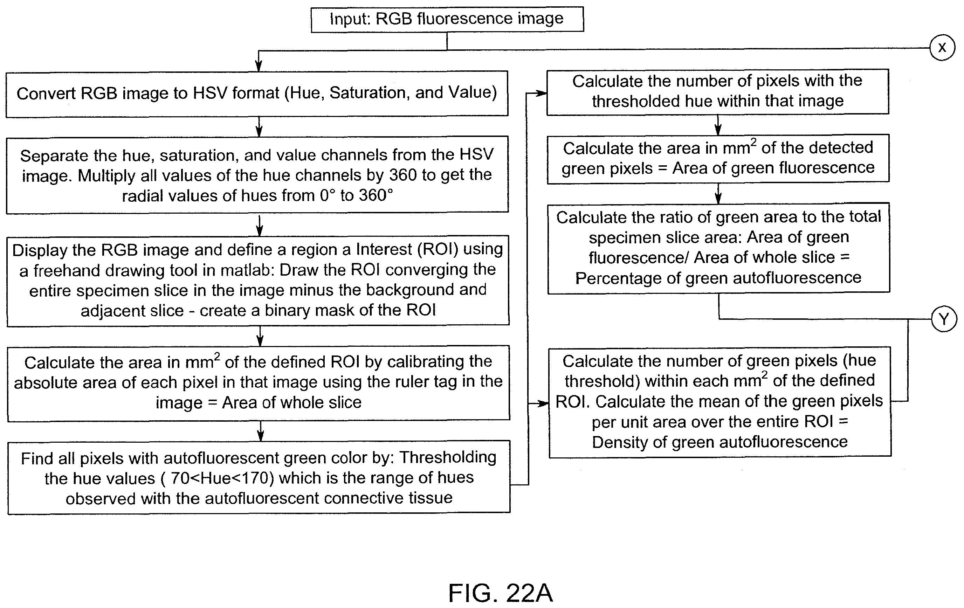

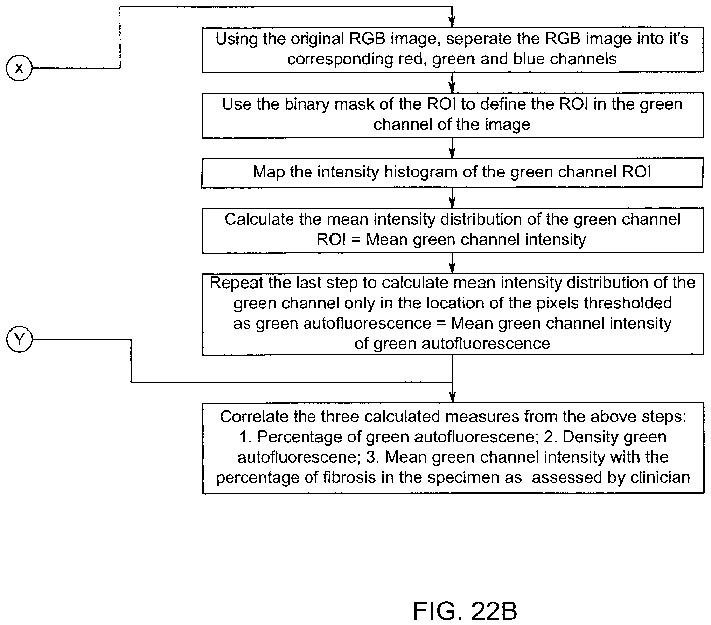

Based on the above observed correlation between the clinician examination of the lumpectomy specimens and the green fluorescence in the imaged tissue, it is possible to utilize such images of breast tissue to predict an amount of fibrosis in the tissues. The flowchart in FIG. 22 describes a method for quantifying the green fluorescence in an image and correlating the amount of green fluorescence in an image to a percentage of fibrosis in a lumpectomy specimen. The custom/proprietary program was run on MATLAB. The method includes determining a percentage of green autofluorescence, density of green autofluorescence, and mean green channel intensity in the image to predict the percentage of fibrosis, as discussed further below. This method can be performed using software running on a handheld imaging device in accordance with the present disclosure or, alternatively, may be performed on a device separate from the imaging device at a later time.

In accordance with the present teachings, a RGB image of interest is input. Next, as shown in blue, the software converts the RGB image to HSV format (Hue, Saturation, and Value). It also contemplated that other color spaces could be used, for example, CMYK and HSL. Those of skill in the art will understand that other color spaces are possible as well. As discussed further, the HSV format may be used to determine the percentage of green autofluorescence and the density of green autofluorescence in the image. The Hue, Saturation, and Value channels are then separated from the HSV image. All values in the Hue channel are multiplied by 360 to obtain radial values of hues from 0 degrees to 360 degrees. On the RGB image, a region of interest (ROI) can be identified using a freehand drawing tool in MATLAB. A user may draw the region of interest, which covers the entire specimen slice in the image minus the background and adjacent slices. The software may then create a binary mask of the region of interest. Next, the software may calculate the area of the region of interest in mm² by calibrating the absolute area of each pixel in that image using the ruler tag in the image in order to determine an Area of the whole slice. The software may then locate all pixels with autofluorescent green color by thresholding the hue values (70<Hue<170), which is the range of hues observed with the autofluorescent connective tissue.

Next, as shown in yellow in FIG. 22, the software may calculate the number of pixels with the thresholded Hue within the image, and calculate the area in mm² of the detected green pixels in order to determine an Area of green fluorescence. Then, the software may calculate a ratio of the green area to the total specimen slice area by calculating a ratio of the Area of green fluorescence with the Area of the whole slice. This ratio provides the percentage of green autofluorescence, which corresponds to the number of pixels in the sample and may be used to determine the percentage of fibrosis in the sample.

As shown in pink in FIG. 22, the system may also calculate the number of green pixels (hue threshold) within each mm² of the defined region of interest. Then, the system may calculate the mean of the green pixels per unit area over the entire region of interest in order to obtain the density of green autofluorescence. The density of green autofluorescence corresponds to the density of the green pixels in the sample and may be used to determine the percentage of fibrosis in the sample.

Alternatively, instead of using HSV, as shown in green in FIG. 22, the inputted RGB fluorescence image may separated into its corresponding Red, Green and Blue channels. The software may then use the binary mask of the region of interest to define the ROI in the Green channel of the image. Next, the software may map the intensity histogram of the green channel region of interest, and calculate the mean intensity distribution of the green channel region of interest in order to determine a mean green channel intensity. Then the software may repeat this last step to calculate mean intensity distribution of the green channel only in the location of the pixels thresholded as green autofluorescence in order to determine the mean green channel intensity of green autofluorescence. The mean green channel intensity of green autofluorescence may correspond to the intensity of the green pixels in the sample and may be used to determine the percentage of fibrosis in the sample.

As shown in gray in FIG. 22, the software may correlate percentage of green autofluorescence, the density of the green autofluorescence, and the mean green channel intensity of green autofluorescence with the percentage of fibrosis in the specimen as assessed by the clinician. Such may be used to predict and determine the percent of fibrosis in a patient in order to provide a proper diagnosis for the patient. For example, women with a higher percentage of fibrosis may have poorer cosmetic outcomes following BCS.

In addition to fibrosis, predictive determinations regarding the composition of tissues or percentages of other types of tissues within the images can be made based on color in the images.

### Color

Images collected by the device are displayed as a composite color image. When imaging is performed in fluorescence mode (405 nm illumination with capture of emitted light in the range of 500-550 nm and 600-660 nm) composite images contain a spectrum of colors resulting from the emission of green light (500 - 550 nm) and red light (600 - 660 nm) or a combination thereof. The wavelength(s) (corresponding to the color) of light emitted from the target are a result of the presence of specific fluorescent molecules. For example, PpIX (a product of 5-ALA metabolism) present in tumors appears red fluorescent while collagen, a component of normal connective tissue, appears green fluorescent. When a mixture of different fluorescent molecules is present in the tissue the resultant color in the composite image is due to a combination of the different emitted wavelengths. The concentration/density and intrinsic fluorescent properties (some fluorescent molecules have stronger intrinsic fluorescent intensity) of each type of fluorescent molecule present in the target tissue will affect the resultant fluorescent color.

Color can be used to assist in classifying the different types of tissues contained within collected images.

### Tissue Classification

Analysis of the fluorescent color (including features such as hue, luminosity, saturation) can provide information about the type and relative amount of different tissue(s) in the target tissue (i.e. what proportion of the target tissue is tumor vs. connective tissue). Luminosity in particular is useful in interpreting fluorescence images, given that tissues with a similar hue can be differentiated visually (and through image analysis) by differences in luminosity. For example, in breast tissue specimens, fat appears pale pink while PplX fluorescent tumors can appear as a range of intensities of red. In some cases, PplX tumor fluorescence will have the same hue as background normal fat tissue, however differences in luminosity will make the PpIX in tumors appear 'more bright'. In addition, subtle differences in color characteristics which are not visually perceptible in the composite images may also be calculated using image analysis software to interpret differences in tissue composition or identify the presence of specific tissue components.

### Image Interpretation

The relationship between fluorescence color and tissue composition allows the user to interpret the composite color image/video (i.e., the user will know what type of tissue he/she is looking at) as well as provides the user with additional information, not otherwise obvious under white light examination, to guide clinical decisions. For example, if the target tissue appears bright red fluorescent to the user (e.g., surgeon), the user will understand that this means there is a high density of tumor cells in that area and may choose to act on the information by removing additional tissue from the surgical cavity. Conversely, if the tissue appears weakly red fluorescent, the user may decide not to remove additional tissue but rather take a small piece of tissue to confirm the presence of tumor microscopically.

Thus, in this sense, the redness of the fluorescence may be considered predictive of tissue type and the presence of disease. In addition to looking at the color contained in the image, the clinician, surgeon, or other medical staff looking at the images may also look at the pattern or "texture" of the image. Further, not only is the intensity of a single color relevant, but combinations of colors together also provide information to the clinician. For example, the identification of green in the image can be an identifier of normal, healthy connective tissue in the image, such as collagen or elastin. The pattern that color makes may also provide an indication regarding the density of the tissue. For example, patchy or mottled green may indicate diffuse connective tissue while solid green may be indicative of dense connective tissue. Similarly, a large solid mass of red may indicate focal tumor or disease, while red dots spread throughout the image may be indicative of multifocal disease.

As noted above, seeing the interaction of or the position of the colors relative to one another can also provide information to the clinician. When red fluorescence and green fluorescence are in an image together, it is possible to see the extent of disease (red fluorescence) within healthy tissue (green fluorescence). Further, the positioning of the red (disease) relative to the green (healthy tissue) can guide a clinician during intervention to remove or resect the disease. Red and green together can also delineate the boundary between diseased and healthy tissue and provide context of the healthy tissue anatomy to guide resection. The combination of these colors together also provides feedback to the surgeon/clinician during Interventions such as resection. That is, as the diseased tissue is removed or otherwise destroyed, the visual representation in red and green will change. As the red disappears and green becomes more prevalent, the surgeon will be receiving affirmative feedback that the disease is being removed, allowing the surgeon to evaluate the effectiveness of the intervention in real-time. This is applicable to many types of image-guided interventions including, for example, laparoscopy, resection, biopsy, curettage, brachytherapy, high-frequency ultrasound ablation, radiofrequency ablation, proton therapy, oncolytic virus, electric field therapy, thermal ablation, photodynamic therapy, radiotherapy, ablation, and/or cryotherapy.

When looking at color/texture/pattern of the image, it is possible for the clinician to differentiate tissue components such as connective tissue, adipose tissue, tumor, and benign tumor (hyperplastic lesions). In one aspect, the clinician can get an overall picture of healthy tissue versus diseased tissue (green versus red), and then, within diseased tissue, potentially differentiate between benign disease and malignant disease based on the intensity of the red fluorescence (benign = weak intensity, malignant = strong intensity). Non-limiting examples of benign disease that may be identified include fibroid adenoma, hyperplasia, lobular carcinoma in situ, adenosis, fat necrosis, papilloma, fibrocystic disease, and mastitis.

Looking at the red and green fluorescence together can also assist clinicians in targeting biopsies and curettage.

When looking at lymph nodes, it may be possible to identify subclinical disease and/or overt disease. The fluorescence image can be used to identify metastatic disease in the lymphatic system, the vascular system, and the interstitial space including infiltrate disease.

Based on the above examples, the features present in a multispectral image can be used to classify tissue and to determine the effectiveness of interventions. In particular, it is possible to do the following using the features found in a multispectral image:
- Classify different tissue types;
- Determine the extent of disease present or absent in imaged tissues;
- Guide sampling of a given area;
- Make diagnoses;
- Make prognoses regarding response to intervention (Fluorescent primary tumor removed? No lymph node involvement? identification of previously unknown lymph node involvement)
- Treatment planning
- Use for guiding treatment (e.g., planting radioactive seeds for prostate cancer, targeting tumor for radiation treatment)
- Predicting disease (density of breast tissue (e.g. collagen))
- Triaging/stratifying patients for treatment, determining follow up treatments based on images

Analysis of the images obtained herein may be performed by software running on the devices described herein or on separate processors. Examples of image analysis and appropriate software may be found, for example, in U.S. Provisional Patent Application No. 62/625, 611, filed February 2, 2018 and entitled "Wound Imaging and Analysis" and in international patent application no. PCT/CA2019/000002 filed on January 15, 2019 and entitled "Wound Imaging and Analysis,"

The multispectral images collected by the devices disclosed in this application, in accordance with the methods described in this application, may lead to the ability to do the following:
1. Multispectral or multiband fluorescence images can be used to differentiate between different tissue components to determine the relative amount of a given tissue component versus other components in an imaging field of view.
2. Multispectral or multiband fluorescence images can be used to qualitatively or quantitatively (for example based on relative fluorescence features) classify healthy vs abnormal tissues as well as classify/characterize various tissue types.
3. Multispectral or multiband fluorescence images can be used for training or education purposes to improve fluorescence image interpretation of a target by users.
4. Multispectral or multiband fluorescence images can be used for training computer algorithms (example machine learning, neural networks, artificial intelligence) to automatically in real time achieve items 1 and 2 above.
5. Multispectral or multiband fluorescence images can be used to determine the concentration (semi-quantitatively) of PpIX.
6. Multispectral or multiband fluorescence images of PpIX fluorescent tumors may be used for dosimetry (e.g. photodynamic therapy) or in planning treatments of tumors,
7. Multispectral or multiband fluorescence images may be used to identify fibrosis in the breast. The pattern, intensity, distribution, diffusivity, etc. of the AF connective tissue is an indicator of fibrosis, rigidity, and tensile strength of tissue. Multispectral or multiband fluorescence images disclosed herein can provide information about the luminosity of a given fluorescent feature, from which a user could differentiate tumor PpIX FL from fat tissue with similar hues but lacking luminescence
8. Multispectral or multiband fluorescence imaging (with 5-ALA) is capable of detecting disease (otherwise clinically occult) which is, for example, about 2.6 mm or less below the imaged tissue surface (see manuscript figure 4).
9. Multispectral or multiband fluorescence images provide color differences between disease and normal tissue so that the boundary between these tissues can be defined and visualized.
10. Multispectral or multiband fluorescence can be used for image-guided biopsy, surgical resection or ablation or cryotherapy.
11. Multispectral or multiband fluorescence can be used for real-time image-guided excision of the primary tumor specimen (e.g. lumpectomy) to minimize the need for additional tissue excision and risk of dissecting the primary tumor. Spatially/anatomically correlating a primary tumor specimen with additional tissue excisions is challenging. Removal of a single specimen may improve the co-registration of a lumpectomy surface with the cavity surface. Furthermore, dissection of the primary tumor may contribute to seeding of tumor cells in the surgical cavity and an increased risk of recurrence.
12. Multispectral or multiband fluorescence images can identify non-tissue components within the field of view (e.g. retractors, surgical/vascular clips, surgical tools) which provides visual feedback of spatial context during imaging and fluorescence-guided resection (e.g. in a darkened room),
13. A method for wherein the multispectral or multiband fluorescence images are used to extract color (e.g. RGB) channels, spectral components (e.g. wavelength histogram) on a pixel, ROI or field of view basis.
14. A method wherein the extracted color channels are arithmetically processed (e.g. ratio of red channel to green channel, artifact/noise reduction, histogram enhancement) to visualize and/or quantify biological features (e.g. structures, concentration differences, tissue differentiation borders, depth) not otherwise perceivable in the raw image.
15. A method wherein the multispectral or multiband fluorescence images can be converted to alternative color spaces (e.g. CIE 1931 Lab space) for the purpose of visualization and/or quantification of biological features (e.g. structures, concentration differences, tissue differentiation borders, depth) not otherwise perceivable in the raw image. For example, by converting into the Lab space colors which were not differentiable in the RGB image but may be differentiable both visually and arithmetically in the Lab space.
16. Multispectral or multiband fluorescence images can be used to quantitatively measure the size, including area, of manual/automatically defined ROIs (e.g. entire specimen, area of green AF) or distance between manually/automatically defined ROIs or tissue differentiation borders (e.g. distance between red fluorescence tumor foci and specimen margin on serially sectioned specimen) at any focal distance within the device's specified focal range.
17. A method wherein a physical or optical calibration tag is placed within the field of view of the camera.

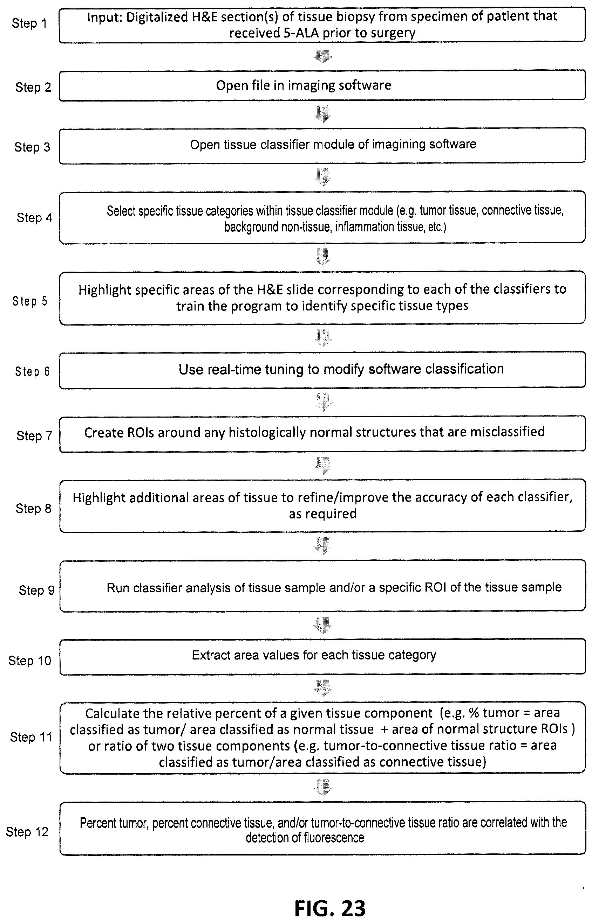

In accordance with another aspect of the present disclosure, a method of quantifying the fluorescence images obtained with the disclosed handheld multispectral device (first method of FIG. 23) is disclosed. In addition, a method of determining the accuracy of the fluorescence images obtained with the disclosed handheld multispectral device (second method of FIG. 23) is also disclosed. The methods are illustrated in the flow chart of FIG. 23. The methods are run, for example, on HALO imaging software. It is also contemplated that other well-known software may be used.

The method of quantifying the fluorescence images (referred to herein as the first method) will be discussed first and will reference various steps identified in FIG. 23. The first method, as shown in FIG. 23, includes the step of inputting in the imaging software digitalized sections of a tissue biopsy of a patient, such that the tissue has been stained with a histological stain, for example, a hematoxylin and eosin stain (H&E stain) and that the patient received 5-ALA prior to surgery (Step 1).

For example, in the first method of FIG. 23, in a patient that received 5-ALA prior to a lumpectomy, a tumor biopsy is first removed from the lumpectomy specimen. The biopsy, for example a core biopsy, is taken from an area which, for example, fluoresced red during imaging with the handheld device, indicating that tissue containing porphyrins (i.e., tumor) is present. One or more portions of the tumor biopsy are then stained with the H&E stain and processed into one or more digital images. As discussed further below, the imaging software analyzes the digital images in order to quantify the tumor biopsy. For example, the software may determine that the tumor biopsy includes 40% tumor tissue, 10% adipose tissue, 10% connective tissue, and 40% other tissue. Such may allow a user to quantify the tumor biopsy by determining the specific amounts of each type of tissue within the biopsy. This allows confirmation that tumor was present in the area(s) that fluoresced red when imaged with the handheld device.

As shown in FIG. 23, in steps 2 and 3 of the first method, a user opens the desired file in the imaging software and then opens, for example, a tissue classifier module in the imaging software. Within the tissue classifier module, one or more specific tissue categories may be selected (step 4). Exemplary tissue categories include, for example, tumor tissue, adipose tissue, connective tissue, background non-tissue, and inflammation tissue. The imaging software will then evaluate the tissue sample based upon the selected tissue category.

As shown in steps 5-8 of FIG. 23, the imaging software may be refined/improved in order to provide a more accurate program. For example, a user may highlight specific areas of the tissue sample stained with H&E corresponding to each of the selected tissue categories (step 5). This may help to train the imaging software to identify specific tissue types. For example a user may highlight connective tissue in the tissue sample stained with H&E in order to help the imaging software identify any and all connective tissue.

The imaging software may also allow a user to modify the imaging software's classification of the tissue sample via real-time tuning. For example, a user may view the imaging software's classification of the tissue sample (step 6). In one example, the imaging software may classify areas in the tissue sample as including connective tissue and the remaining areas as being background non-tissue. The user may then create a region of interest (ROI) around any histologically normal structures that are misclassified (step 7). For example, the user may identify one or more portions of the areas classified as connective tissue that are actually background non-tissue. Thus, the user may identify one or more areas in which the imaging device misclassified the portions as connective tissue. Such an identification may be used to refine/improve the imaging device in order to improve its accuracy in correctly identifying tissue. The user may also highlight additional areas of interest in the tissue sample in order to further refine/improve the accuracy of each tissue category (step 8).

In step 9 of the first method of FIG. 23, a user may run, within the imaging software, the tissue classifier module. Therefore, the imaging software may analyze the digital image (of the tissue stained with, for example, H&E) in order to quantify the different tissue components. As discussed above, such may allow a user to determine the different tissue components in the tumor biopsy. Thus, for example, a tumor biopsy may be removed from a an excised tissue specimen. One or more portions of the tumor biopsy may be stained with the H&E stain and processed into the digital images. These one or more portions may be based upon the detected areas of the fluorescent emissions from the disclosed multispectral device. For example, a portion of the tumor biopsy having a larger percent of red fluorescent (cancer tissue) may be processed for the digital section images. The software (in step 9 of FIG. 23) may then analyze the digital images in order to determine the specific tissue components (and their quantity) within the portion of the tumor biopsy. Thus, the software may determine that the portion of the tumor having a larger percent of red fluorescent has more than an average amount of adipose tissue. By quantifying the different tissue compenents in the tumor biopsy, a user may be better able to study and understand the tumor (for example, how the tumor was affecting the heath of the patient).

It is also contemplated that the imaging device may perform the analysis in step 9 (for the first method) on only a specific portion of the tissue sample, for example, on a specific region of interest within the tissue sample. In some embodiments, the region of interest may be a particular area of the tissue sample that is, for example, about one-third in size of the total tissue sample. In other embodiments, the region of interest may be an area of the tissue sample that is within a specific distance from the imaged surface.

The imaging software may extract area values (e.g. mm²) for each of the selected tissue categories (step 10) of FIG. 23. For example, for the first method, the software may determine the area values of each of the selected tissue categories in the tissue sample, The software may then calculate the relative percent of a specific tissue component in the tissue sample (step 11).

In the second method of FIG. 23, and as discussed further below, the imaging software may also be used to compare tissue detected by the H&E stain with that detected by the disclosed multispectral device. The imaging software may then determine the accuracy of the disclosed multispectral device based upon this comparison. In this case, the type of sample used for the digital image sections may be different. For example, instead of taking a core sample, a whole mount process may be used. This permits a one-to-one or pixel-by-pixel comparision between the stained tissue sample and the tissue sample that was imaged using the handheld imaging device. For example, the imaging software compares, in the same tissue sample, the connective tissue stained pink with the H&E stain and the green autofluorescence detected by the disclosed multispectral device. As discussed above, the presence and amount of green autofluorescence may represent the presence and amount of connective tissue in the tissue sample. The imaging software may then determine the accuracy of the disclosed multispectral device by comparing the pink stain (from the H&E stain) with the green autofluorescence (from the disclosed multispectral device),

A method of determining the accuracy of the fluorescence images obtained with the disclosed handheld multispectral device (second method of FIG. 23) will now be discussed with reference various steps identified in FIG. 23. The second method, as shown in FIG. 23, includes the step of inputting in the imaging software digitalized sections of a tissue biopsy of an excised tissue specimen, such as a lumpectomy tissue specimen removed during breast cancer surgery. As noted above, a whole mount staining may be used. The digitalized tissue sections are of tissue that been stained with a histological stain, for example, a hematoxylin and eosin stain (H&E stain). Further, the digitalized tissue sections are of the biopsies taken from the tissue imaged with the handheld imaging device disclosed herein, wherein the patient received 5-ALA prior to surgery (Step 1).

As shown in FIG. 23, in steps 2 and 3 of the second method, a user opens the desired file in the imaging software and then opens, for example, a tissue classifier module in the imaging software. Within the tissue classifier module, one or more specific tissue categories may be selected (step 4). Exemplary tissue categories include, for example, tumor tissue, adipose tissue, connective tissue, background non-tissue, and inflammation tissue. The imaging software will then evaluate the tissue sample based upon the selected tissue category.

As shown in steps 5-8 of FIG. 23, the imaging software may be refined/improved in order to provide a more accurate program. For example, a user may highlight specific areas of the tissue sample stained with H&E corresponding to each of the selected tissue categories (step 5). This may help to train the imaging software to identify specific tissue types. For example a user may highlight connective tissue in the tissue sample stained with H&E in order to help the imaging software identify any and all connective tissue.

The imaging software may also allow a user to modify the imaging software's classification of the tissue sample via real-time tuning. For example, a user may view the imaging software's classification of the tissue sample (step 6). In one example, the imaging software may classify areas in the tissue sample as including connective tissue and the remaining areas as being background non-tissue. The user may then create a region of interest (ROI) around any histologically normal structures that are misclassified (step 7). For example, the user may identify one or more portions of the areas classified as connective tissue that are actually background non-tissue. Thus, the user may identify one or more areas in which the imaging device misclassified the portions as connective tissue. Such an identification may be used to refinelimprove the imaging device in order to improve its accuracy in correctly identifying tissue. The user may also highlight additional areas of interest in the tissue sample in order to further refine/improve the accuracy of each tissue category (step 8).

In step 9 of the second method of FIG. 23, the software may compare the tissue sample with regard to the H&E stain and with regard to the green autofluorescence, within the context of the selected tissue categories. In this method, the software compares the tissue samples in order to determine the accuracy of the fluorescence images obtained with the disclosed handheld multispectral device. n one example, if a user selects the tissue category of connective tissue, the amount of connective tissue detected by the software in the H&E stained tissue is compared with the amount of connective tissue detected by the software in the fluorescent tissue (in step 9 of FIG. 23). This comparison is then used to determine if the disclosed handheld multispectral device adequately captured the connective tissue in the tissue sample., or said another way, if the amount of fluorescence of a given color, which is understood to correspond to a particular tissue type, can be correlated by determining the tissue types in the same sample (in a pixel by pixel analysis) when stained with the H&E stain,

It is also contemplated that the imaging device may perform the analysis in step 9 (for the second method) on only a specific portion of the tissue sample, for example, on a specific region of interest within the tissue sample. In some embodiments, the region of interest may be a particular area of the tissue sample that is, for example, about one-third in size of the total tissue sample. In other embodiments, the region of interest may be an area of the tissue sample that is within a specific distance from the imaged surface.

The imaging software may extract area values (e.g. mm²) for each of the selected tissue categories (step 10) in the second method of FIG. 23. For the second method, the imaging software may calculate a first area value for the connective tissue identified with the H&E stain and a second area value for the connective tissue identified with the disclosed multispectral device. The imaging software may further calculate a third area value for the tumor tissue identified with the H&E stain and a fourth area value for the tumor tissue identified with the disclosed multispectral device. Using the calculated area values, the imaging software may then determine the accuracy of the disclosed multispectral device (step 11). For example, the imaging software may use the first and second area values to determine the percent of connective tissue identified by the H&E stain and identified by the disclosed multispectral device. Thus, the imaging software may, for example, determine that the H&E stain shows that the tissue sample includes 45% connective tissue and that the disclosed multispectral device shows that the tissue sample include 45% connective tissue. In this example, the imaging software may then determine that the disclosed multispectral device is accurate in its determination of identifying connective tissue (because the first area value is equal to the second area value).

In another example, the imaging software may determine, for example, that the H&E stain shows that the tissue sample includes 35% connective tissue while the disclosed multispectral device shows that the tissue sample include 25% connective tissue. In this example, the imaging software may then determine that the multispectral device is not accurate in its determination of identifying connective tissue and needs refinement, or that the imaging software itself needs refinement in is determination of identifying connective tissue (because the first area value is not equal to the second area value).

In order to determine the percent of each tissue category in the tissue sample, the imaging device may use the area values, as discussed above. For example, in order to calculate the relative percentage of a given tissue category, the imaging device may divide the area value of that tissue category by the area classified as normal tissue. The area classified as normal tissue may also include any region of interest specifically identified by the user as being normal tissue, as discussed above.

The imaging device may also use the area values, as discussed above, to determine a ratio of two components. For example, to determine a ratio of tumor tissue to connective tissue. Thus, the imaging device may divide the area value of the tissue classified as tumor tissue with the area value of the tissue classified as connective tissue.

As discussed above, the data from the H&E stain is compared/correlated with the fluorescence images (step 12). This may be used to to determine the accuracy of the disclosed multispectral device). Thus, a user may determine that the multispectral device accurately detects the presence and amount of tumor tissue but fails to accurately detect the presence and/or amount of connective tissue. Such may be helpful to refine the multispectral device.

The disclosed multispectral device may be refined by altering the optical filter of the device. For example, the transmission band of the optical filter may be varied in order to alter the detected fluorescence. Such may allow, for example, less green fluorescence to be viewed, which may more accurately correlate to the actual presence of connective tissue in the biopsy.

In some embodiments, the disclosed imaging device may be used with adipose tissue that produces, for example, a pinkish brown fluorescence emission. In this example, a user would select the tissue category of adipose tissue. In other embodiments, tissue categories such as blood and abnormal tissue (e.g., tumor, cancerous cells, lesions, benign tumor, and hyperplastic lesions) may be selected.

After a first tissue category is selected, a user may then select a second tissue category. The imaging software would then create a new first area value and a new second area value for the second tissue category. The software may then compare the new first area value and the new second are value, as discussed above with regard to the first and second area values.

Is it also contemplated that the disclosed imaging software allows a user to determine if the multispectral device needs refinement without a high level of expertise by the user. Thus, the imaging device provides an easy and automated system to determine if the multispectral device needs refinement.

The imaging software can be used with other devices other than the disclosed multispectral device. Thus, the imaging device may be used with a variety of devices in order to determine the accuracy of the device, and whether it needs refinement.

It is contemplated that the steps of FIG. 23 may be interchanged and applied in another order than disclosed herein. Additionally, one or more steps may be omitted.

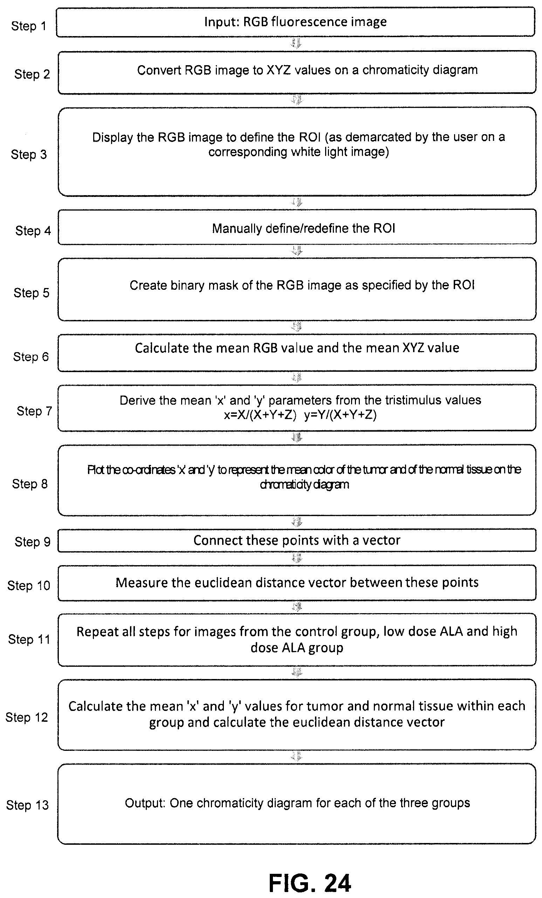

In accordance with another aspect of the present disclosure, a method of quantifying color contrast is disclosed. For example, the method may be used to quantify the fluorescence color contrast between tumor tissue and normal tissue. Thus, the average color intensity of the tumor tissue is compared with the average color intensity of the normal tissue. In some embodiments, the method may be used to quantify the fluorescence color contrast between different intensities of connective tissue. Thus, the average color intensity of a first area of the connective tissue is compared with the average color intensity of a second area of the connective tissue. Such color contrasts may not be reliable when perceived with a user's eye. For example, both the first and second areas may have a green autofluorescence that is so similar, a user's eye may not be able to discern the difference in color between these two areas. Thus, the method of FIG. 24 provides an accurate process to identify such color contrasts. The method of FIG. 24 may also be used to quantify color contrast with the H&E stained tissue samples.

The method is illustrated in the flow chart of FIG. 24. The method can be run on proprietary/custom software using, for example, MATLAB software. It is also contemplated that other well-known softwares may be used in place of MATLAB.

As shown in step 1 of FIG. 24, the method includes inputting into the imaging software an RGB image, for example, an RGB fluorescence image. Thus, the RGB fluorescence image may be an image of a tissue sample that includes green and/or red fluorescence, as discussed above. Next, in step 2, the imaging software may convert the RGB image into a data set to obtain tristimulus values for the image. For example, the imaging software may convert the RGB image into XYZ values on a chromaticity diagram (CIE color system) in to order to provide a spatial location of each pixel in the RGB image.

The imaging software may also display the region of interest (ROI) in the tissue sample (step 3). For example, the region of interest may be demarcated by the user on a corresponding white light image of the tissue. The imaging software may then display this same region of interest in the RGB image. In one example, the region of interest may be a specific area that includes a high level of connective tissue or tumor tissue. In another example, the region of interest may include both tumor tissue and normal tissue. It is also contemplated that more than one region of interest may be used.

As shown in Step 4 of FIG. 24, a user may manually define/redefine the region of interest with a freehand drawing tool on the imaging software. Such may allow a user to modify and tailor the region of interest for a specific application.

In step 5, the imaging software may create a binary mask of the RGB image. As discussed further below, the binary mask may be used to determine the XYV values from the RGB image. The binary mask may be created for only the area(s) specified by the region of interest. Next, the imaging software may calculate a mean RGB value and a mean XYZ value (step 6). For example, the imaging software may create a mean RGB value on a green fluorescence portion of the connective tissue and a corresponding XYZ value. The mean value may be, for example, an average green intensity in the region of interest, and the mean XYV value may be, for example, a corresponding tristimulus value.

Next, in step 7, the imaging software may derive the mean 'x' and 'y' parameters from the tristimulus values calculated in step 6. The 'x' value may be calculated according to the following formula: x=X/(X+Y+Z), and the 'y' value may be calculated according to the following formula: y=Y/(X+Y+Z). In step 8, a user may plot the 'x' and 'y' co-ordinates on a chromaticity diagram to represent the mean color of the specified tissue sample. For example, the specified tissue sample may have a green fluorescence color with a wavelength of 520 nm on the chromaticity diagram.

In some embodiments, the imaging software may create two 'x' and 'y' coordinates on the chromaticity diagram. The two coordinates may originate from the same tissue sample such that one coordinate correlates to tumor tissue and the other coordinate correlates to normal tissue. In other embodiments, one coordinate may correlate to tumor tissue in a first area of the tumor and the other coordinate correlates to tumor tissue in a second area of the same tumor,

As shown in step 9, the imaging software may then connect the two coordinates with a vector. In one example, a first coordinate has a wavelength of 520 nm (green) and a second coordinate has a wavelength of 640 nm (red) on the chromaticity diagram (so that the coordinates represent healthy and tumor tissue, respectively). A vector may connect these two coordinates. Then, in step 10, the imaging software may measure the Euclidean distance vector between the first and second coordinates. The Euclidean distance vector may provide an indication as to the color contrast between the green and red fluorescence colors in the RGB image. Thus, the Euclidean distance vector provides a method/system to quantify the color contrast between the green (normal tissue) and red (tumor tissue). Such may allow a user to easily determine the normal tissue in the specimen compared to the healthy tissue. Additionally, such may allow a user to quantify the difference. A larger difference may be indicative of tumor tissue with a higher density, whereas a smaller difference may be indicative of a tumor tissue with a lower density. Additionally or alternatively, a larger difference may be indicative of a higher dose of ALA in the patient.

In some embodiments, both the first and second coordinates may represent tumor tissue. Thus, the first coordinate may have a wavelength of 640 nm on the chromaticity diagram and the second coordinate may have a wavelength of 700 nm on the chromaticity diagram. Therefore, the second coordinate may correlate to tissue that has a darker red appearance than the first coordinate. The Euclidean distance vector between these two coordinates may allow a user to confirm that a color contrast does indeed exist between the two samples (which may be hard to ascertain based upon a user's vision alone). More specifically, the Euclidean distance vector may confirm that the two tissue samples are indeed different shades of red. Additionally, based upon the Euclidean distance vector, the imaging software may determine that the tissue sample with the darker shade of red (the second coordinate) has a higher density of tumor cells than the tissue sample with the lighter shade of red (the first coordinate). Such may allow a user to quantitively determine the relative densities of tumor cells In one or more specified areas. In some examples, the tissue sample with the lighter shade of red may correspond to benign tissue, while the tissue sample with the darker shade of red may correspond to malignant tissue. Thus, the imaging system may allow a user to quantitively determine whether a tissue sample is benign or malignant.

As shown in step 11 of FIG, 24, the method may further include repeating all of the above steps for a control group, a low dose ALA group, and a high dose ALA group. Then, the imaging software may then calculate mean 'x' and 'y' values for, as an example, tumor and normal tissue with each group, as discussed above (step 12). The imaging software may then calculate the Euclidean distance vector between the mean tumor tissue and mean normal tissue, as discussed above.

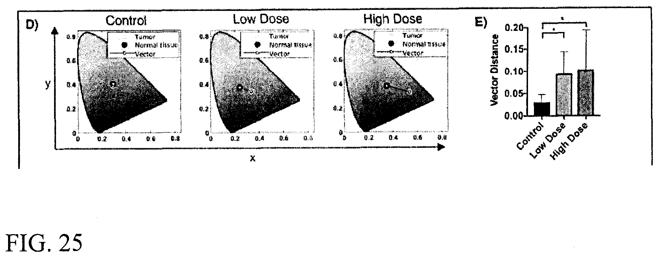

In step 13, the imaging system may output a chromaticity diagram for each of the three groups (control group, low dose ALA, and high dose ALA), as shown in FIG. 25. Each chromaticity diagram may include two points connected by a vector that depicts the distance between mean tumor color and mean normal tissue color within each group. A user may then compare the chromaticity diagrams for the three groups to quantitively assess the differences.

It is contemplated that the steps of FIG. 24 may be interchanged and applied in another order than disclosed herein. Additionally, one or more steps may be omitted.

It will be appreciated by those ordinarily skilled in the art having the benefit of this disclosure that the present disclosure provides various exemplary devices, systems, and methods for intraoperative or ex *vivo* visualization of tumors and/or residual cancer cells on surgical margins. Further modifications and alternative embodiments of various aspects of the present disclosure will be apparent to those skilled in the art in view of this description.

Furthermore, the devices and methods may include additional components or steps that were omitted from the drawings for clarity of illustration and/or operation, Accordingly, this description is to be construed as illustrative only and is for the purpose of teaching those skilled in the art the general manner of carrying out the present disclosure. It is to be understood that the various embodiments shown and described herein are to be taken as exemplary. Elements and materials, and arrangements of those elements and materials, may be substituted for those illustrated and described herein, parts and processes may be reversed, and certain features of the present disclosure may be utilized independently, all as would be apparent to one skilled in the art after having the benefit of the description herein. Changes may be made in the elements described herein without departing from the spirit and scope of the present disclosure and following claims, including their equivalents.
It is to be understood that the particular examples and embodiments set forth herein are non-limiting, and modifications to structure, dimensions, materials, and methodologies may be made without departing from the scope of the present disclosure.

Furthermore, this description's terminology is not intended to limit the present disclosure. For example, spatially relative terms-such as "beneath," "below," "lower," "above," "upper," "bottom," "right," "left," "proximal," "distal," "front," and the like-may be used to describe one element's or feature's relationship to another element or feature as illustrated in the figures. These spatially relative terms are intended to encompass different positions (i.e., locations) and orientations (i.e., rotational placements) of a device in use or operation in addition to the position and orientation shown in the drawings.

For the purposes of this specification and appended claims, unless otherwise indicated, all numbers expressing quantities, percentages or proportions, and other numerical values used in the specification and claims, are to be understood as being modified in all instances by the term "about" if they are not already. Accordingly, unless indicated to the contrary, the numerical parameters set forth in the following specification and attached claims are approximations that may vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques.

Notwithstanding that the numerical ranges and parameters setting forth the broad scope of the present disclosure are approximations, the numerical values set forth in the specific examples are reported as precisely as possible. Any numerical value, however, inherently contains certain errors necessarily resulting from the standard deviation found in their respective testing measurements. Moreover, all ranges disclosed herein are to be understood to encompass any and all sub-ranges subsumed therein.

It is noted that, as used in this specification and the appended claims, the singular forms "a," "an," and "the," and any singular use of any word, include plural referents unless expressly and unequivocally limited to one referent. As used herein, the term "include" and its grammatical variants are intended to be non-limiting, such that recitation of items in a list is not to the exclusion of other like items that can be substituted or added to the listed items.

It should be understood that while the present disclosure has been described in detail with respect to various exemplary embodiments thereof, it should not be considered limited to such, as numerous modifications are possible without departing from the scope of the appended claims.

## Claims

1. A handheld, white light and fluorescence-based imaging device (600; 700; 800; 900; 1000; 1100) for visualizing at least one of precancerous cells, cancerous cells, and satellite lesions in surgical margins, comprising:
a body (610; 710; 810; 910; 1010; 1110) having a first end portion (112) configured to be held in a user's hand and a second end portion (114), wherein the second end portion (114) of the body (610; 710; 810; 910; 1010; 1110) is elongated and configured to:
be at least partially positioned within a surgical cavity containing a surgical margin,
be inserted through a surgical incision of 3 cm in size and into the surgical cavity,
direct light onto the surgical margin,
wherein the body (610; 710; 810; 910; 1010; 1110) contains:
at least one excitation light source configured to excite autofluorescence emissions of tissue cells and fluorescence emissions of induced porphyrins in tissue cells of the surgical margin;
a filter (652; 752; 852; 952; 1052; 1152) configured to prevent passage of reflected excitation light and permit passage of emissions having a wavelength corresponding to autofluorescence emissions of tissue cells and fluorescence emissions of the induced porphyrins in tissue cells;
an imaging lens (662; 762; 862; 962; 1062; 1162);
an image sensor (648; 748; 848; 948; 1048; 1148) configured to detect the filtered autofluorescence emissions of tissue cells and fluorescence emissions of the induced porphyrins in tissue cells of the surgical margin;
a processor configured to receive the detected emissions and to output data regarding the detected filtered autofluorescence emissions of tissue cells and fluorescence emissions of the induced porphyrins in tissue cells of the surgical margin; and
inductive charging coils (640; 740; 840; 940; 1040; 1140) for charging the device.

2. The device of claim 1, wherein a distal end portion is configured to be positioned adjacent to the surgical margin without contacting the surgical margin.

3. The device of claim 1 or claim 2, wherein the at least one excitation light source emits light having a wavelength of between about 375 nm and about 800 nm.

4. The device of any one of claims 1-3, wherein the at least one excitation light source includes a first excitation light source that emits a first excitation light having a wavelength between about 375 nm and about 430 nm and a second excitation light source that emits a second excitation light having a wavelength between about 550 nm and about 600 nm.

5. The device of any one of claims 1-4, wherein:
the at least one excitation light source includes a first light source (120) comprising a plurality of LEDs configured to emit light at a first wavelength;the at least one excitation light source includes a second light source (124) comprising a second plurality of LEDs configured to emit light at a second wavelength, different from the first wavelength;
the at least one excitation light source further includes a third excitation light source (128) that emits light at a third wavelength, different from the first wavelength and the second wavelength;
the third excitation light source (128) is positioned adjacent to the first and second excitation light sources (120, 124); and
the third excitation light source (128) is configured to excite tissue cells of the surgical margin that contain near-infrared or infrared dye.

6. The device of claim 5, wherein the third wavelength is about 700 nm to about 750 nm, about 750 nm to about 800 nm, or about 800 nm to about 850 nm.

7. The device of any one of claims 1-6, further comprising a white light source to facilitate white light imaging of the surgical cavity.

8. The device of any of clams 1-7, further comprising controls for at least one of power on/power off, image mode/video mode, excitation light/white light, and filter on/filter off.

9. The device of any of claims 1-8, further comprising an ambient light sensor configured to indicate when fluorescence imaging conditions are appropriate.

10. The device of any one of claims 1-9, wherein the device is configured to wirelessly transmit the data regarding the detected filtered autofluorescence emissions of tissue cells and fluorescence emissions of the induced porphyrins in tissue cells of the surgical margin.

11. The device of any one of claims 1-10, wherein the device is configured to detect cancerous cells containing porphyrins induced via administration of a therapeutic or a diagnostic dosage of a non-activated, non-targeted compound configured to induce porphyrins in cancerous tissue.

12. The device of any one of claims 1-11, wherein the first end portion (112) of the body (610; 710; 810; 910; 1010; 1110) of the device forms a base of the device, and wherein the inductive charging coils (640; 740; 840; 940; 1040; 1140) are positioned in the base of the device for wireless charging of the device.

13. A multispectral system (1300) for visualizing cancerous cells in surgical margins, comprising:
a handheld device (600; 700; 800; 900; 1000; 1100) according to any one of claims 1-12;
a display device (1280) configured to display data output by the processor of the handheld device (600; 700; 800; 900; 1000; 1100); and
a wireless real-time data storage and pre-processing device, optionally
wherein the wireless real-time data storage and pre-processing device is configured to receive video and/or image data transmitted from the handheld device (600; 700; 800; 900; 1000; 1100).

14. The system of claim 13, further comprising a sterilization case configured to receive the handheld device (600; 700; 800; 900; 1000; 1100) and/or a charging dock for wirelessly charging the handheld device (600; 700; 800; 900; 1000; 1100) .

15. A kit for white light and fluorescence-based visualization of cancerous cells in a surgical margin, comprising:
a handheld device (600; 700; 800; 900; 1000; 1100) according to any one of claims 1-12; and
a non-targeted, non-activated compound configured to induce porphyrins in cancerous tissue cells;
optionally wherein the non-targeted, non-activated compound is configured to be administered topically, orally, intravenously, via aerosol, via immersion, and/or via lavage.

16. The kit of claim 15, wherein the non-targeted, non-activated compound is aminolevulinic acid.

17. The kit of claim 15, further comprising a drape to cover the device and provide a sterile barrier, wherein the drape optionally includes or is coupled with an optical window that covers a distal end of the handheld device to ensure accurate transmission of light emitted from the device.

18. A method of assessing an excised tissue specimen, comprising:
subsequent to the administration to the excised tissue specimen of a non-activated, non-targeted compound configured to induce porphyrins in cancerous tissue cells, and with the device of any one of claims 1-12:
illuminating tissue cells of the excised tissue specimen with an excitation light;
detecting fluorescence emissions from tissue cells in the excised tissue specimen that contain induced porphyrins; and
displaying in real-time the tissue cells from which fluorescence emissions were detected.

## Patentansprüche

1. Handgeführte, auf Weißlicht und Fluoreszenz basierende
Bildgebungsvorrichtung (600; 700; 800; 900; 1000; 1100) zum Visualisieren von wenigstens einem aus präkanzerösen Zellen, kanzerösen Zellen und Satellitenläsionen in chirurgischen Rändern, umfassend:
einen Körper (610; 710; 810; 910; 1010; 1110), welcher einen ersten Endabschnitt (112), welcher dazu eingerichtet ist, in einer Hand eines Benutzers gehalten zu werden, und einen zweiten Endabschnitt (114) aufweist, wobei der zweite Endabschnitt (114) des Körpers (610; 710; 810; 910; 1010; 1110) länglich ist und dazu eingerichtet ist:
wenigstens teilweise innerhalb einer chirurgischen Höhle positioniert zu sein, welche einen chirurgischen Rand enthält,
durch einen chirurgischen Schnitt mit einer Größe von 3 cm und in die chirurgische Höhle eingeführt zu werden;
Licht auf den chirurgischen Rand zu richten,
wobei der Körper (610; 710; 810; 910; 1010; 1110) enthält:
wenigstens eine Anregungslichtquelle, welche dazu eingerichtet ist, Autofluoreszenzemissionen von Gewebezellen und Fluoreszenzemissionen von induzierten Porphyrinen in Gewebezellen des chirurgischen Rands anzuregen;
einen Filter (652; 752; 852; 952; 1052; 1152), welcher dazu eingerichtet ist, einen Durchgang von reflektiertem Anregungslicht zu verhindern und einen Durchgang von Emissionen zuzulassen, welche eine Wellenlänge aufweisen, die Autofluoreszenzemissionen von Gewebezellen und Fluoreszenzemissionen der induzierten Porphyrine in Gewebezellen entspricht;
eine Bildgebungslinse (662; 762; 862; 962; 1062; 1162);
einen Bildsensor (648; 748; 848; 948; 1048; 1148), welcher dazu eingerichtet ist, die gefilterten Autofluoreszenzemissionen von Gewebezellen und Fluoreszenzemissionen der induzierten Porphyrine in Gewebezellen des chirurgischen Rands zu detektieren;
einen Prozessor, welcher dazu eingerichtet ist, die detektierten Emissionen zu empfangen und Daten in Bezug auf die detektierten gefilterten Autofluoreszenzemissionen von Gewebezellen und Fluoreszenzemissionen der induzierten Porphyrine in Gewebezellen des chirurgischen Rands auszugeben; und
induktive Ladespulen (640; 740; 840; 940; 1040; 1140) zum Laden der Vorrichtung.

2. Vorrichtung nach Anspruch 1, wobei ein distaler Endabschnitt dazu eingerichtet ist, benachbart zu dem chirurgischen Rand positioniert zu sein, ohne den chirurgischen Rand zu kontaktieren.

3. Vorrichtung nach Anspruch 1 oder 2, wobei die wenigstens eine Anregungslichtquelle Licht emittiert, welches eine Wellenlänge von zwischen etwa 375 nm und etwa 800 nm aufweist.

4. Vorrichtung nach einem der Ansprüche 1-3, wobei die wenigstens eine Anregungslichtquelle eine erste Anregungslichtquelle, welche ein erstes Anregungslicht emittiert, welches eine Wellenlänge zwischen etwa 375 nm und etwa 430 nm aufweist, und eine zweite Anregungslichtquelle umfasst, welche ein zweites Anregungslicht emittiert, welches eine Wellenlänge zwischen etwa 550 nm und etwa 600 nm aufweist.

5. Vorrichtung nach einem der Ansprüche 1-4, wobei:
die wenigstens eine Anregungslichtquelle eine erste Lichtquelle (120) umfasst, welche eine Mehrzahl von LEDs umfasst, die dazu eingerichtet sind, Licht mit einer ersten Wellenlänge zu emittieren;
die wenigstens eine Anregungslichtquelle eine zweite Lichtquelle (124) umfasst, welche eine zweite Mehrzahl von LEDs umfasst, die dazu eingerichtet sind, Licht mit einer zweiten Wellenlänge zu emittieren, welche von der ersten Wellenlänge verschieden ist;
die wenigstens eine Anregungslichtquelle ferner eine dritte Anregungslichtquelle (128) umfasst, welche Licht mit einer dritten Wellenlänge emittiert, welche von der ersten Wellenlänge und der zweiten Wellenlänge verschieden ist;
die dritte Anregungslichtquelle (128) benachbart zu der ersten und der zweiten Anregungslichtquelle (120, 124) positioniert ist; und
die dritte Anregungslichtquelle (128) dazu eingerichtet ist, Gewebezellen des chirurgischen Rands anzuregen, welche Nahinfrarot- oder Infrarotfarbstoff enthalten.

6. Vorrichtung nach Anspruch 5, wobei die dritte Wellenlänge etwa 700 nm bis etwa 750 nm, etwa 750 nm bis etwa 800 nm oder etwa 800 nm bis etwa 850 nm beträgt.

7. Vorrichtung nach einem der Ansprüche 1-6, ferner umfassend eine Weißlichtquelle, um eine Weißlichtbildgebung der chirurgischen Höhle zu erleichtern.

8. Vorrichtung nach einem der Ansprüche 1-7, ferner umfassend Steuerungen für wenigstens eines aus Einschalten/Ausschalten, Bildmodus/Videomodus, Anregungslicht/Weißlicht und Filter ein/Filter aus.

9. Vorrichtung nach einem der Ansprüche 1-8, ferner umfassend einen Umgebungslichtsensor, welcher dazu eingerichtet ist, anzuzeigen, wenn Fluoreszenzbildgebungsbedingungen geeignet sind.

10. Vorrichtung nach einem der Ansprüche 1-9, wobei die Vorrichtung dazu eingerichtet ist, Daten in Bezug auf die detektierten gefilterten Autofluoreszenzemissionen von Gewebezellen und Fluoreszenzemissionen der induzierten Porphyrine in Gewebezellen des chirurgischen Rands drahtlos zu übertragen.

11. Vorrichtung nach einem der Ansprüche 1-10, wobei die Vorrichtung dazu eingerichtet ist, kanzeröse Zellen zu detektieren, welche Porphyrine enthalten, die über eine Verabreichung einer therapeutischen oder einer diagnostischen Dosis einer nicht aktivierten, nicht zielgerichteten Verbindung induziert worden sind, welche dazu eingerichtet ist, Porphyrine in kanzeröses Gewebe zu induzieren.

12. Vorrichtung nach einem der Ansprüche 1-11, wobei der erste Endabschnitt (112) des Körpers (610; 710; 810; 910; 1010; 1110) der Vorrichtung eine Basis der Vorrichtung bildet und wobei die induktiven Ladespulen (640; 740; 840; 940; 1040; 1140) zum drahtlosen Laden der Vorrichtung in der Basis der Vorrichtung positioniert sind.

13. Multispektrales System (1300) zum Visualisieren von kanzerösen Zellen in chirurgischen Rändern, umfassend:
eine handgeführte Vorrichtung (600; 700; 800; 900, 1000; 1100) nach einem der Ansprüche 1-12;
eine Anzeigevorrichtung (1280), welche dazu eingerichtet ist, durch den Prozessor der handgeführten Vorrichtung (600; 700; 800; 900, 1000; 1100) ausgegebene Daten anzuzeigen; und
eine drahtlose Echtzeit-Datenspeicher- und Vorverarbeitungsvorrichtung, optional wobei die drahtlose Echtzeit-Datenspeicher- und Vorverarbeitungsvorrichtung dazu eingerichtet ist, von der handgeführten Vorrichtung (600; 700; 800; 900, 1000; 1100) übertragene Video- und/oder Bilddaten zu empfangen.

14. System nach Anspruch 13, ferner umfassend einen Sterilisationsbehälter, welcher dazu eingerichtet ist, die handgeführte Vorrichtung (600; 700; 800; 900, 1000; 1100) aufzunehmen, und/oder eine Ladestation zum drahtlosen Laden der handgeführten Vorrichtung (600; 700; 800; 900, 1000; 1100).

15. Kit für eine auf Weißlicht und Fluoreszenz basierende Visualisierung von kanzerösen Zellen in chirurgischen Rändern, umfassend:
eine handgeführte Vorrichtung (600; 700; 800; 900, 1000; 1100) nach einem der Ansprüche 1-12; und
eine nicht zielgerichtete, nicht aktivierte Verbindung, welche dazu eingerichtet ist, Porphyrine in kanzeröse Gewebezellen zu induzieren;
optional wobei die nicht zielgerichtete, nicht aktivierte Verbindung dazu eingerichtet ist, topisch, oral, intravenös, über Aerosol, über Immersion und/oder über Lavage verabreicht zu werden.

16. Kit nach Anspruch 15, wobei die nicht zielgerichtete, nicht aktivierte Verbindung Aminolävulinsäure ist.

17. Kit nach Anspruch 15, ferner umfassend ein Drape, um die Vorrichtung abzudecken und eine sterile Barriere bereitzustellen, wobei das Drape optional ein optisches Fenster umfasst oder damit gekoppelt ist, welches ein distales Ende der handgeführten Vorrichtung abdeckt, um eine genaue Übertragung von Licht sicherzustellen, welches von der Vorrichtung emittiert wird.

18. Verfahren zum Beurteilen einer exzidierten Gewebeprobe, umfassend:
auf die Verabreichung einer nicht aktivierten, nicht zielgerichteten Verbindung, welche dazu eingerichtet ist, Porphyrine in kanzeröse Gewebezellen zu induzieren, an die exzidierte Gewebeprobe folgend und mit der Vorrichtung nach einem der Ansprüche 1-12:
Beleuchten von Gewebezellen der exzidierten Gewebeprobe mit einem Anregungslicht;
Detektieren von Fluoreszenzemissionen von Gewebezellen in der exzidierten Gewebeprobe, welche induzierte Porphyrine enthalten; und
Anzeigen der Gewebezellen, von welchen Fluoreszenzemissionen detektiert worden sind, in Echtzeit.

## Revendications

1. Dispositif d'imagerie portatif (600 ; 700 ; 800 ; 900 ; 1000 ; 1100), à base de lumière blanche et de fluorescence pour visualiser au moins l'une parmi des cellules précancéreuses, des cellules cancéreuses, et des lésions satellites dans des marges chirurgicales, comprenant :
un corps (610 ; 710 ; 810 ; 910 ; 1010 ; 1110) ayant une première portion d'extrémité (112) configurée pour être maintenue dans une main d'un utilisateur et une seconde portion d'extrémité (114), dans lequel la seconde portion d'extrémité (114) du corps (610 ; 710 ; 810 ; 910 ; 1010 ; 1110) est allongée et configurée pour :
être au moins partiellement positionnée à l'intérieur d'une cavité chirurgicale contenant une marge chirurgicale,
être insérée à travers une incision chirurgicale de 3 cm de taille et dans la cavité chirurgicale,
diriger de la lumière sur la marge chirurgicale,
dans lequel le corps (610 ; 710 ; 810 ; 910 ; 1010 ; 1110) contient :
au moins une source de lumière d'excitation configurée pour exciter des émissions d'autofluorescence des cellules tissulaires et des émissions de fluorescence de porphyrines induites dans des cellules tissulaires de la marge chirurgicale ;
un filtre (652 ; 752 ; 852 ; 952 ; 1052 ; 1152) configuré pour empêcher un passage de la lumière d'excitation reflétée et permettre un passage d'émissions ayant une longueur d'onde correspondant aux émissions d'autofluorescence des cellules tissulaires et des émissions de fluorescence des porphyrines induites dans des cellules tissulaires ;
une lentille d'imagerie (662 ; 762 ; 862 ; 962 ; 1062 ; 1162) ;
un capteur d'image (648 ; 748 ; 848 ; 948 ; 1048 ; 1148) configuré pour détecter les émissions d'autofluorescence filtrées des cellules tissulaires et des émissions de fluorescence des porphyrines induites dans des cellules tissulaires de la marge chirurgicale ;
un processeur configuré pour recevoir les émissions détectées et pour délivrer des données concernant les émissions d'autofluorescence filtrées détectées des cellules tissulaires et des émissions de fluorescence des porphyrines induites dans des cellules tissulaires de la marge chirurgicale ; et
des bobines de chargement inductif (640 ; 740 ; 840 ; 940 ; 1040 ; 1140) pour charger le dispositif.

2. Dispositif selon la revendication 1, dans lequel une portion d'extrémité distale est configurée pour être positionnée adjacente à la marge chirurgicale sans contacter la marge chirurgicale.

3. Dispositif selon la revendication 1 ou la revendication 2, dans lequel la au moins une source de lumière d'excitation émet de la lumière ayant une longueur d'onde comprise entre environ 375 nm et environ 800 nm.

4. Dispositif selon l'une quelconque des revendications 1 à 3, dans lequel la au moins une source de lumière d'excitation inclut une première source de lumière d'excitation qui émet une première lumière d'excitation ayant une longueur d'onde comprise entre environ 375 nm et environ 430 nm et une seconde source de lumière d'excitation qui émet une seconde lumière d'excitation ayant une longueur d'onde comprise entre environ 550 nm et environ 600 nm.

5. Dispositif selon l'une quelconque des revendications 1 à 4, dans lequel :
la au moins une source de lumière d'excitation inclut une première source de lumière (120) comprenant une pluralité de LED configurées pour émettre de la lumière à une première longueur d'onde ;
la au moins une source de lumière d'excitation inclut une seconde source de lumière (124) comprenant une seconde pluralité de LED configurées pour émettre de la lumière à une seconde longueur d'onde, différente de la première longueur d'onde ;
la au moins une source de lumière d'excitation inclut en outre une troisième source de lumière d'excitation (128) qui émet de la lumière à une troisième longueur d'onde, différente de la première longueur d'onde et de la seconde longueur d'onde ;
la troisième source de lumière d'excitation (128) est positionnée adjacente à la première et à la seconde source de lumière d'excitation (120, 124) ; et
la troisième source de lumière d'excitation (128) est configurée pour exciter des cellules tissulaires de la marge chirurgicale qui contiennent un colorant du proche-infrarouge ou infrarouge.

6. Dispositif selon la revendication 5, dans lequel la troisième longueur d'onde est d'environ 700 nm à environ 750 nm, d'environ 750 nm à environ 800 nm, ou d'environ 800 nm à environ 850 nm.

7. Dispositif selon l'une quelconque des revendications 1 à 6, comprenant en outre une source de lumière blanche pour faciliter l'imagerie par lumière blanche de la cavité chirurgicale.

8. Dispositif selon l'une quelconque des revendications 1 à 7, comprenant en outre des commandes pour au moins l'un d'une mise en marche/arrêt, d'un mode imagerie/mode vidéo, d'une lumière d'excitation/lumière blanche, et d'un filtre en marche/filtre en arrêt.

9. Dispositif selon l'une quelconque des revendications 1 à 8, comprenant en outre un capteur de lumière ambiante configuré pour indiquer le moment où des conditions d'imagerie par fluorescence sont appropriées.

10. Dispositif selon l'une quelconque des revendications 1 à 9, dans lequel le dispositif est configuré pour transmettre sans fil les données concernant les émissions d'autofluorescence filtrées détectées des cellules tissulaires et des émissions de fluorescence des porphyrines induites dans des cellules tissulaires de la marge chirurgicale.

11. Dispositif selon l'une quelconque des revendications 1 à 10, dans lequel le dispositif est configuré pour détecter des cellules cancéreuses contenant des porphyrines induites par l'intermédiaire d'une administration d'une dose thérapeutique ou de diagnostic d'un composé non activé, non ciblé configuré pour induire des porphyrines dans du tissu cancéreux.

12. Dispositif selon l'une quelconque des revendications 1 à 11, dans lequel la première portion d'extrémité (112) du corps (610 ; 710 ; 810 ; 910 ; 1010 ; 1110) du dispositif forme une base du dispositif, et dans lequel les bobines de chargement inductif (640 ; 740 ; 840 ; 940 ; 1040 ; 1140) sont positionnées dans la base du dispositif pour le chargement sans fil du dispositif.

13. Système multispectral (1300) pour visualiser des cellules cancéreuses dans des marges chirurgicales, comprenant :
un dispositif portatif (600 ; 700 ; 800 ; 900 ; 1000 ; 1100) selon l'une quelconque des revendications 1 à 12 ;
un dispositif d'affichage (1280) configuré pour afficher une sortie de données par le processeur du dispositif portatif (600 ; 700 ; 800 ; 900 ; 1000 ; 1100) ; et
un dispositif de stockage et de prétraitement de données en temps réel et sans fil, éventuellement dans lequel le dispositif de stockage et de prétraitement de données en temps réel et sans fil est configuré pour recevoir des données vidéo et/ou d'image transmises depuis le dispositif portatif (600 ; 700 ; 800 ; 900 ; 1000 ; 1100).

14. Système selon la revendication 13, comprenant en outre un boîtier de stérilisation configuré pour recevoir le dispositif portatif (600 ; 700 ; 800 ; 900 ; 1000 ; 1100) et/ou une station de chargement pour charger sans fil le dispositif portatif (600 ; 700 ; 800 ; 900 ; 1000 ; 1100).

15. Kit de visualisation basée sur de la lumière blanche et la fluorescence de cellules cancéreuses dans une marge chirurgicale, comprenant :
un dispositif portatif (600 ; 700 ; 800 ; 900 ; 1000 ; 1100) selon l'une quelconque des revendications 1 à 12 ; et
un composé non ciblé, non activé configuré pour induire des porphyrines dans des cellules tissulaires cancéreuses ;
éventuellement dans lequel le composé non ciblé, non activé est configuré pour être administré par voie topique, orale, intraveineuse, par l'intermédiaire d'un aérosol, par immersion, et/ou par lavage.

16. Kit selon la revendication 15, dans lequel le composé non ciblé, non activé est l'acide aminolévulinique.

17. Kit selon la revendication 15, comprenant en outre un champ pour recouvrir le dispositif et fournir une barrière stérile, éventuellement dans lequel le champ inclut ou est accouplé à une fenêtre optique qui recouvre une extrémité distale du dispositif portatif pour garantir une transmission exacte de la lumière émise depuis le dispositif.

18. Procédé d'évaluation d'un échantillon de tissu excisé, comprenant les étapes consistant à :
ultérieurement à l'administration à l'échantillon de tissu excisé d'un composé non activé, non ciblé configuré pour induire des porphyrines dans des cellules tissulaires cancéreuses, et avec le dispositif selon l'une quelconque des revendications 1 à 12 :
éclairer des cellules tissulaires de l'échantillon de tissu excisé avec de la lumière d'excitation ;
détecter des émissions de fluorescence des cellules tissulaires dans l'échantillon de tissu excisé qui contiennent des porphyrines induites ; et
afficher en temps réel les cellules tissulaires à partir desquelles les émissions de fluorescence étaient détectées.
